# EUROPÄISCHE PATENTSCHRIFT

(11) **EP 3 331 581 B1**
(45) Veröffentlichungstag und Bekanntmachung des Hinweises auf die Patenterteilung: **08.07.2020**
(21) Anmeldenummer: 16747513.6
(22) Anmeldetag: 03.08.2016
(51) Int. Cl.: A61L 27/28, A61L 27/50, A61L 29/14, A61L 31/08, A61L 31/14, A61B 17/02

(54) **MECHANOPHORES MEDIZINPRODUKT**
MECHANOPHORIC MEDICAL PRODUKT
PRODUIT MEDICAL MECANOPHORIQUE

(30) Priorität: 07.08.2015 DE 102015215112; 29.02.2016 DE 102016203287
(43) Veröffentlichungstag der Anmeldung: 13.06.2018
(73) Patentinhaber: Aesculap AG, 78532 Tuttlingen (DE)
(72) Erfinder: BROSIG, Kris, 73655 Plüderhausen (DE); UTZ, Michael, 78532 Tuttlingen (DE)
(74) Vertreter: Patentanwälte Ruff, Wilhelm, Beier, Dauster & Partner mbB
(86) Internationale Anmeldenummer: PCT/EP2016/068540
(87) Internationale Veröffentlichungsnummer: WO 2017/025408

(56) Entgegenhaltungen:
- EP-A2- 2 237 014
- WO-A1-2011/082287
- WO-A1-2012/054140
- WO-A1-2013/088172
- WO-A1-2013/158379
- WO-A1-2016/022661
- WO-A2-2007/005401
- WO-A2-2012/125736
- DE-A1-102013 005 874
- DE-U1- 20 320 501
- US-A1- 2003 216 732
- US-A1- 2004 064 191
- US-A1- 2008 074 643
- US-A1- 2014 013 864
- YING JIANG: "An outlook review: Mechanochromic materials and their potential for biological and healthcare applications", MATERIALS SCIENCE AND ENGINEERING C., Bd. 45, 14. August 2014 (2014-08-14), Seiten 682-689, XP055307607, CH ISSN: 0928-4931, DOI: 10.1016/j.msec.2014.08.027
- DATABASE WPI Week 201573 Thomson Scientific, London, GB; AN 2015-63025K XP002762614, & CN 104 880 820 A (UNIV SOUTHEAST) 2. September 2015 (2015-09-02)

## Beschreibung

### ANWENDUNGSGEBIET UND STAND DER TECHNIK

Die Erfindung betrifft ein Medizinprodukt, welches ein Mechanophor enthält. Beschrieben wird die Verwendung eines Mechanophors zur Herstellung eines Medizinprodukts.

Polymere sind heute fast allgegenwärtig: Von Verpackungen bis zu Spezialprodukten in der Medizintechnik. Die Polymere sind dabei Belastungen ausgesetzt, welche zu einer Schädigung der Polymerstruktur und mithin zu einem Material- sowie Produktversagen führen können. Der Detektion und Lokalisation von Material- oder Produktzonen, welche anwendungsbedingt besonders hohen Belastungen ausgesetzt sind, kommt daher eine besondere Bedeutung zu. Eine Methode zur Detektion von Materialschäden ist aus der WO 2006/105290 A2 bekannt. Die PCT-Offenlegungsschrift schlägt die Verwendung von farbigen Substanzen vor, welche in einer Matrix in Form von Kapseln integriert sind. Anfänglich ist die Farbe der Substanzen nicht sichtbar. Erst durch Schädigung der Kapseln werden die farbigen Substanzen freigesetzt.

Aus der WO 2007/003883 A1 ist die Verwendung von elektromagnetischen Fasern bekannt, welche bei Beschädigung u.a. lumineszierende Eigenschaften annehmen können.

Ein weiterer Ansatz besteht in der Verwendung von sogenannten tribolumineszierenden Materialien. Hierbei handelt es sich um Materialien, welche bei starker mechanischer Belastung eine "kalte Lichtemission" zeigen. Entsprechende Materialien sind beispielsweise aus der US 7,242,443 B2 bekannt.

Mechanophorhaltige Polymere sowie mechanophorhaltige Zusammensetzungen sind aus der WO 2009/018111 A1 sowie US 2014/0013864 A1 bekannt. Unter einem Mechanophor versteht man eine Verbindung, welche bei Belastung eine chemisch und/oder physikalisch detektierbare Strukturänderung, wie beispielsweise Farbänderung, erfährt.

Mechanophorhaltige Polymere sind auch in der Nichtpatentliteratur beschrieben: Beiermann et al.: "Role of Mechanophor Orientation in Mechanochemical Reactions" ACS Macro Lett. 2012, 1, 163-166; Kim et al.: "Mechanoactivation of Spiropyran Covalently Linked PMMA: Effect of Temperature, Strain Rate, and Deformation Mode", Macromolecules 2015, 48, 1335-1342; Beiermann et al.: "Environmental Effects on Mechanochemical Activation of Spiropyran in Linear PMMA" J. Mater. Chem., 2011, 21, 8443-8447; Kean et al.: "Stress-Responsive Polymers Containing Cyclobutane Core Mechanophores: Reactivity and Mechanistic Insights" J. Am. Chem. Soc. 2013, 135, 13598-13604; Kingsbury et al.: "Shear Activation of Mechanophore-Crosslinked Polymers" J. Mater. Chem., 2011 21, 8381-8388 sowie Brown und Craig: "Molecular Engineering of Mechanophore Activity for Stress-Responsive Polymeric Materials" Chem. Sci., 2015, 6, 2158-2165.

Der Detektion von Material- bzw. Produktversagen kommt auch auf dem Gebiet der Medizintechnik eine besondere Bedeutung zu. Dies gilt insbesondere auf dem Gebiet der Arthroplastik, d.h. für chirurgische Eingriffe, welche die Gelenkfunktionen sicher- oder wiederherstellen sollen. So muss bei der Auswahl und Positionierung eines diesbezüglich geeigneten Implantats stets die Bandsituation der Gelenke sowie die Lastverteilung berücksichtigt werden.

Beispielsweise darf bei der Durchführung einer vollständigen Kniearthroplastie (Total Knee Arthroplasty, TKA) die Last nicht ungleich auf das mediale und laterale Kompartiment des Gelenks verteilt sein. Ferner ist zu beachten, dass die Bandspannung nicht zu gering ist. Andererseits darf die Bandspannung aber auch nicht zu groß sein, um eine Luxation, einen erhöhten Abrieb sowie eine eingeschränkte Beweglichkeit zu vermeiden. Der Operateur muss daher eine passende Implantatposition sowie eine geeignete Gleitflächenhöhe auswählen.

Ein ähnliches Problem stellt sich bei der Durchführung einer vollständigen Hüftarthroplastie (Total Hip Arthroplasty, THA). Auch hier muss die Bandspannung am Gelenk anhand unterschiedlicher Implantatvarianten sowie Kopf-/Halslängen möglichst optimal eingestellt werden. Die Beurteilung einer korrekten Bandspannung sowie vor allem einer gleichmäßigen Lastverteilung stellt auch in diesem Fall eine große Herausforderung dar.

Bislang erfolgt die Beurteilung der Bandspannung in der Regel taktil durch den Arzt. Dabei werden Probeimplantatrepositionen vorgenommen, um die Gelenkstabilität zu beurteilen. Nachteilig ist, dass es sich hierbei um eine ausschließlich subjektive Beurteilung der Bandspannung handelt.

Für die Kniearthroplastik kann mittlerweile auch auf eine elektronische Systemlösung zurückgegriffen werden, welche insbesondere eine Beurteilung der Druckverteilung von medialem und lateralem Kompartiment ermöglicht. Nachteilig ist, dass solche Systemlösungen einen erheblichen Investitionsaufwand erfordern.

WO2013088172 beschreibt orthopädische Implantate wie Gelenkprothesen, mit Lagern die deformierbare Abschnitte mit kapazitiven Sensorelementen enthalten.

### AUFGABE UND LÖSUNG

Der vorliegenden Erfindung liegt daher die Aufgabe zugrunde, ein Medizinprodukt bereitzustellen, welches eine möglichst zuverlässige und insbesondere objektive Beurteilung, bevorzugt intraoperative Beurteilung, von im Körper eines menschlichen oder tierischen Patienten auftretenden Kräften und insbesondere von Kräfteverteilungen im menschlichen oder tierischen Körper erlaubt.

Diese Aufgabe wird erfindungsgemäß gelöst durch ein Medizinprodukt mit den Merkmalen des unabhängigen Anspruchs 1. Bevorzugte Ausführungsformen sind in den abhängigen Ansprüchen definiert.

Die Erfindung betrifft ein Medizinprodukt, welches ein Mechanophor enthält, wobei es sich bei dem Medizinprodukt um ein Implantat oder Implantatteil handelt, dadurch gekennzeichnet, dass es sich bei dem Implantat oder Implantatteil um eine Gelenkprothese, ein Teil einer Gelenkprothese, ein Probeimplantat oder ein Probeimplantatteil handelt.

Unter dem Ausdruck "Mechanophor" soll im Sinne der vorliegenden Erfindung eine Verbindung gemäß eingangs erwähnter Definition verstanden werden, d.h. eine Verbindung, welche bei Belastung eine chemisch und/oder physikalisch detektierbare Strukturänderung erfährt.

Unter dem Ausdruck "Belastung" soll im Sinne der vorliegenden Erfindung vorzugsweise eine mechanische Belastung, insbesondere eine Zug-, Druck- und/oder Scherbelastung, verstanden werden.

Durch die bei Belastung nachweisbare Strukturänderung des Mechanophors ist es möglich, Kräfte und insbesondere Kräfteverteilungen zu erfassen und zu beurteilen, welche im Körper eines menschlichen oder tierischen Patienten auf das Medizinprodukt und/oder einen Behandlungssitus, welcher mit dem Medizinprodukt versorgt werden soll, einwirken. Eine Auswertung derartiger Kräfte bzw. Kraftverteilungen kann beispielsweise über ein 3D-Mikroskop oder ein Endoskop erfolgen. Insbesondere kann eine Auswertung von Verformungen des Medizinprodukts über digitale Bildverarbeitung automatisiert erfolgen.

Das erfindungsgemäße Medizinprodukt erlaubt vor allem eine intraoperative Beurteilung solcher Kräfte bzw. Kräfteverteilungen, insbesondere bei der Durchführung von Implantationen. Dies ist vor allem bei der Durchführung von arthroplastischen Eingriffen von Vorteil, wo zur Vermeidung von Band- und/oder Sehnenschädigungen eine möglichst exakte Lastindikation sowie ein möglichst exaktes Aufzeigen der Lastverteilung, insbesondere in künstlichen Gelenken, erforderlich sind. Das erfindungsgemäße Medizinprodukt trägt somit zu einer wesentlichen Verbesserung des medizinischen Behandlungserfolges, vorzugsweise Implantationserfolges, sowie zu einer reduzierten Revisionsrate, insbesondere auf dem Gebiet der Arthroplastik, bei.

In einer bevorzugten Ausführungsform ist das Mechanophor Bestandteil einer Beschichtung des Medizinprodukts bzw. in einer Beschichtung des Medizinprodukts enthalten. Mit anderen Worten ist das Mechanophor gemäß einer bevorzugten Ausführungsform Bestandteil einer Schicht des Medizinprodukts bzw. in einer Schicht des Medizinprodukts enthalten. Die Beschichtung bzw. Schicht ist vorzugsweise auf der Oberfläche (Außenoberfläche) des Medizinprodukts ausgebildet.

In einer bevorzugten Ausführungsform ist das Mechanophor Bestandteil einer Mischung oder Zusammensetzung des Medizinprodukts.

Bei der Mischung bzw. Zusammensetzung handelt es sich in einer weiteren Ausführungsform um eine Beschichtung des Medizinprodukts. Anders ausgedrückt, bildet die Mischung bzw. Zusammensetzung gemäß einer weiteren Ausführungsform eine Schicht des medizinischen Produkts. Die Beschichtung bzw. Schicht ist vorzugsweise auf der Oberfläche (Außenoberfläche) des Medizinprodukts ausgebildet.

In einer alternativen Ausführungsform handelt es sich bei der Mischung bzw. Zusammensetzung um ein Vollmaterial des Medizinprodukts. Anders ausgedrückt, besteht das Medizinprodukt gemäß einer alternativen Ausführungsform aus der Mischung bzw. Zusammensetzung.

Das Medizinprodukt, insbesondere die Beschichtung oder die Mischung bzw. Zusammensetzung, weist in einer weiteren Ausführungsform ferner ein Polymer auf.

Das Polymer ist vorzugsweise ausgewählt aus der Gruppe aufweisend oder bestehend aus Polyolefine, Polyethylen (PE), ultrahochmolekulares Polyethylen (UHMWPE), Polyvinylchlorid (PVC), Polyvinylidenchlorid, Polyvinylfluorid, Polyvinylidenfluorid, Polytetrafluorethylen (PTFE), Polystyrol, Polyvinylalkohol (PVA), Polyacrylsäure oder Polyacrylat, Polymethacrylsäure oder Polymethacrylat, Polymethylmethacrylsäure oder Polymethylmethacrylat, Polydimethylsiloxan, Polyoxymethylen (POM), Polyoxyethylen, Polyurethane, amorphe Polymere, Elastomere, thermoplastische Elastomere, Polypropylen, Polytetrafluorpropylen, Polyhexafluorpropylen, Polyethylenglykol, Polypropylenglykol, Polyester, Polyethylenterephthalsäure oder Polyethylenterephthalat, Polypropylenterephthalsäure oder Polypropylenterephthalat, Polybutylenterephthalsäure oder Polybutylenterephthalat, Polyhydroxyalkansäuren oder Polyhydroxyalkanoate, Polymilchsäure bzw. Polylactid, Polyglykolsäure oder Polyglykolat, Polyhydroxybuttersäure oder Polyhydroxybutyrat, Poly-3-hydroxybuttersäure oder Poly-3-hydroxybutyrat, Poly-4-hydroxybuttersäure oder Poly-4-hydroxybutyrat, Polytrimethylencarbonat, Poly-e-caprolacton, Poly-para-Dioxanon, Copolymere, insbesondere Di- oder Terpolymere, und Mischungen, insbesondere Blends, davon.

Das Mechanophor ist in einer weiteren Ausführungsform kovalent mit dem Medizinprodukt, insbesondere Polymer, verknüpft.

Insbesondere kann das Mechanophor kovalent in die Hauptkette (molekulares Rückgrat) des Polymers und/oder kovalent in eine Seitenkette des Polymers integriert sein und/oder eine Seitenkette des Polymers bilden.

Weiterhin können Moleküle des Polymers über das Mechanophor miteinander vernetzt sein.

Gemäß einer alternativen Ausführungsform ist das Mechanophor nicht kovalent mit dem Medizinprodukt, insbesondere Polymer, verknüpft.

In einer bevorzugten Ausführungsform erfährt das Mechanophor bei Belastung eine sichtbare Strukturänderung. Auf diese Weise können die zu beurteilenden Kräfte visualisiert werden.

Besonders bevorzugt handelt es sich bei dem Mechanophor um eine mechanochrome Verbindung, d.h. um eine Verbindung, welche bei Belastung eine farbverursachende oder farbverändernde, insbesondere farbton-, farbsättigungs- und/oder farbhelligkeitsverändernde, Strukturänderung erfährt. Dadurch ist es möglich, durch Auftreten von Färbungen oder Farbveränderungen, insbesondere Farbton-, Farbsättigungs- und/oder Farbhelligkeitsveränderungen, Kräfte und insbesondere Kraftverteilungen zu erfassen und zu beurteilen.

Die Strukturänderung geht vorzugsweise mit einer Ringöffnung des Mechanophors einher.

Erfindungsgemäß kann es weiterhin bevorzugt sein, dass die Strukturänderung des Mechanophors reversibel ist, insbesondere unter Einwirkung von Licht, vorzugsweise UV-Licht. Auf diese Weise ist eine mehrfache Verwendung des Medizinprodukts möglich.

In einer weitergehenden Ausführungsform ist das Mechanophor ausgewählt aus der Gruppe aufweisend oder bestehend aus Pyranverbindungen, Oxazinverbindungen, Fulgidverbindungen, Fulgimidverbindungen, dimere Lactonverbindungen, dimere Imidazolverbindungen, Oxicamverbindungen, Piroxicam, Indandionverbindungen, 2,2-bis[4-Dimethylamino)phenyl]-1,3-Indandion, Bicycloverbindungen und Mischungen davon.

Die Pyranverbindungen können ausgewählt sein aus der Gruppe aufweisend oder bestehend aus Naphthopyranverbindungen, Naphtho[1,2-b]pyranverbindungen, Naphtho[2,1-b]pyranverbindungen, Indenokondesiertes Naphthopyranverbindungen, heterozyklischkondensiertes Naphthopyranverbindungen, Spiro-9-Fluoreno[1,2-b]pyranverbindungen, Phenanthropyranverbindungen, Quinolinpyranverbindungen, Fluoroanthenopyranverbindungen, Spiropyranverbindungen, Spiro[benzindolin]naphthopyranverbindungen, Spiro(indolin)benzopyranverbindungen, Spiro(indolin)naphthopyranverbindungen, Spiro(indolin)quinolinpyranverbindungen, Spiro(indolin)pyranverbindungen und Mischungen davon.

Die Pyranverbindungen können insbesondere ausgewählt sein aus der Gruppe aufweisend oder bestehend aus 12-Ethoxy-3-(2,4-dimethoxyphenyl)-3-(4-methoxyphenyl)-3H-benzo[H]pyrano[3,2-c]quinolin, 12-Ethoxy-3-(2-fluorophenyl)-3-(4-methoxy)-3H-benzo[H]pyrano[3,2-c]quinolin, 12-Ethoxy-3,3-diphenyl-3H-benzo[H]pyrano[3,2-c]quinolin, 2-(2,4-Dimethoxyphenyl)-5-ethoxy-9-methoxy-2-(4-methoxyphenyl)-2H-pyrano[3,2-c]quinolin, 2-(4-(3-Dimethylaminopropyl)methylaminophenyl)-2-phenyl-5-methoxycarbonyl-6-methyl-9-methoxy-2H-naphtho[1,2-b]pyran, 2-(4-Bis(3-dimethylaminopropyl)aminophenyl)2-phenyl-5-methoxycarbonyl-6-methyl-9-methoxy-2H-naphtho[1,2-b]pyran, 2-(4-Methoxyphenyl)-2-(4-morpholinophenyl)-5-hydroxy-6-carboethoxy-2H-naphtha[1,2-b]pyran, 2-(4-Methoxyphenyl)-2-phenyl-5-methoxycarbonyl-8,9-dimethoxy-2H-naphtho[1,2-b]pyran, 2-(4-Methoxyphenyl)-2-phenyl-5-morpholino-6-carbomethoxy-9-methoxy-2H-naphtho[1,2-b]pyran, 2-(4-Methoxyphenyl)-2-phenyl-5-morpholino-6-carbomethoxy-9-methyl-2H-naphtho[1,2-b]pyran, 2,2,5-Triphenyl-6-carboethoxy-2H-naphtho[1,2-b]pyran, 2,2,7,7-Tetraphenyl-4-oxo-2,3,4,7-tetrahydro-1H-[1,3]oxazino[5',4':3,4]naphtho[1,2-b]pyran, 2,2,7,7-Tetraphenyl-4-oxo-2,3,4,7-tetrahydro-1H-pyrimidino[5',4':3,4]naphtho[1,2-b]pyran, 2,2-Di(4-methoxyphenyl)-5-methoxycarbonyl-6-(3,4-dimethoxyphenyl)-8,9-dimethoxy-2H-naphtho[1,2-b]pyran, 2,2-Di(4-methoxyphenyl)-5-methoxycarbonyl-6-methyl-8,9-dimethoxy-2H-naphtho[1,2-b]pyran, 2,2-Di(4-methoxyphenyl)-5-methoxycarbonyl-6-phenyl-8,9-dimethoxy-2H-naphtho[1,2-b]pyran, 2,2-Di(4-methoxyphenyl)-5-methoxycarbonyl-6-phenyl-8-morpholino-9-methoxy-2H-naphtho[1,2-b]pyran, 2,2-Di(4-methoxyphenyl)-5-methoxycarbonyl-6-phenyl-8-piperidino-9-methoxy-2H-naphtho[1,2-b]pyran, 2,2-Di(4-methoxyphenyl)-5-methoxycarbonyl-6-phenyl-9,10-dihydro-2H-[1,4]dioxino[2',3':8,9]naphtho[1,2-b]pyran, 2,2-Di-(4-methoxyphenyl)-5-methoxycarbonyl-8,9-dimethoxy-2H-naphtho[1,2-b]pyran, 2,2-Diphenyl-5-hydroxy-6-carbomethoxy-9-methoxy-2H-naphtho[1,2-b]pyran, 2,2-Diphenyl-5-methoxycarbonyl-6-(3,4-dimethoxyphenyl)-8,9-dimethoxy-2H-naphtho[1,2-b]pyran, 2,2-Diphenyl-5-methoxycarbonyl-6-phenyl-2H-[1,3]dioxolo[4',5':8,9]naphtho[1,2-b]pyran, 2,2-Diphenyl-5-methoxycarbonyl-8,9-dimethoxy-2H-naphtho[1,2-b]pyran, 2,2-Diphenyl-6-methyl-5,6-dihydro-2H-pyrano[3,2-c]quinolin-5-on, 2,2-Phenyl-5-hydroxy-6-carboethoxy-2H-naphtho[1,2-b]pyran, 2,2-Phenyl-5-hydroxy-6-morpholinocarbonyl-2H-naphtho[1,2-b]pyran, 2,2-Phenyl-5-methoxy-6-carboethoxy-2H-naphtho[1,2-b]pyran, 2,2-Phenyl-5-morpholino-6-carboethoxy-2H-naphtho[1,2-b]pyran, 2,7,7-Triphenyl-4-oxo-4,7-dihydro[1,3]oxazino[5',6:3,4]naphtho[1,2-b]pyran, 2,7,7-Triphenyl-4-oxo-4H-7H-[1,3]dioxino[5',4':3,4]naphtho[1,2-b]pyran, 2-Phenyl-7-(4-methoxyphenyl)-7-(4-morpholinophenyl)-4-oxo-4,7-dihydro[1,3]oxazino[5',6:3,4]naphtha[1,2-b]pyran, 2-Phenyl-7,7-di(4-methoxyphenyl)-4-oxo-4,7-dihydro[1,3]oxazino[5',6:3,4]naphtha[1,2-b]pyran, 2-Propyl-7,7-di(4-methoxyphenyl)-4-oxo-4,7-dihydro[1,3]oxazino[5',6:3,4]naphtha[1,2-b]pyran, 3 -(4-Morpholinophenyl)-3-phenyl-6,11-difluoro-13,13-dimethyl-3H,13H-indeno[2',3' :3,4]naphtho[1,2-b]pyran, 3-(2-Ethoxycarbonylethyl)-7,7-diphenyl-2,4dioxo-2,3,4,7-tetrahydro[1,3]oxazino[5',6':3,4]naphtho[1,2-b]pyran, 3-(2-Methacryloyloxyethyl)-7,7-diphenyl-2,4-dioxo-2,3,4,7-tetrahydro[1 ,3]oxazino[5',6':3,4]naphtho[1,2-b]pyran, 3-(4-Bis(3-dimethylaminopropyl)aminophenyl)3H-naphtho[2,1-b]pyran, 3-(4-Fluorophenyl)-3-(4-(2-methylpiperidino)phenyl)-13,13-dimethyl-3H,13H-indeno[2',3':3,4]naphtho[1,2-b]pyran, 3-(4-Fluorophenyl)-3-(4-(N,N-diethylamino)phenyl)-13,13-dimethyl-3H,13H-indeno[2',3':3,4]naphtho[1,2-b]pyran, 3-(4-Fluorophenyl)-3-(4-methoxyphenyl)-6,11-dichloro-13,13-dimethyl-3H,13H-indeno[2',3':3,4]naphtho[1,2-b]pyran, 3-(4-Fluorophenyl)-3-(4-methoxyphenyl)-6,11-difluoro-13,13-dimethyl-3H,13H-indeno[2',3':3,4]naphtho[1,2-b]pyran, 3-(4-Fluorophenyl)-3-(4-methoxyphenyl)-6-methoxy-7-(4-(2-methacryloxyethyl)carbamyloxypiperidin-1-yl)-13,13-dimethyl-3H,13H-indeno[2',3':3,4]naphtho[1,2-b]pyran, 3-(4-Fluorophenyl)-3-(4-morpholinophenyl)-13,13-dimethyl-3H,13H-indeno[2',3':3,4]naphtho[1,2-b]pyran, 3-(4-Fluorophenyl)-3-(4-morpholinophenyl)-6,11-difluoro-13,13-dimethyl-3H,13H-indeno[2',3':3,4]naphtho[1,2-b]pyran, 3-(4-Fluorophenyl)-3-(4-morpholinophenyl)-6-methoxy-7-(4-(2-methacryloxyethyl)carbamyloxypiperidin-1-yl)-13,13-dimethyl-3H,13H-indeno[2',3':3,4]naphtho[1,2-b]pyran, 3-(4-Fluorophenyl)-3-(4-piperazinophenyl)-13,13-dimethyl-3H,13H-indeno[2',3':3,4]naphtho[1,2-b]pyran, 3-(4-Fluorophenyl)-3-(4-piperidinophenyl)-13,13-dimethyl-3H,13H-indeno[2',3':3,4]naphtho[1,2-b]pyran, 3-(4-Fluorophenyl)-3-(4-piperidinophenyl)-6,11-difluoro-13,13-dimethyl-3H,13H-indeno[2',3':3,4]naphtho[1,2-b]pyran, 3-(4-Fluorophenyl)-3-(4-pyrrolidinophenyl)-13,13-dimethyl-3H,13H-indeno[2',3':3,4]naphtho[1,2-b]pyran, 3-(4-Fluorophenyl)-3-ferrocenyl-13,13-dimethyl-3H,13H-indeno[2',3':3,4]naphtho[1 ,2-b]pyran, 3-(4-Fluorophenyl)-3-ferrocenyl-6,7-dimethoxy-11-phenyl-13,13-dimethyl-3H,13H-indeno[2',3':3,4]naphtho[1,2-b]pyran, 3-(4-Fluorophenyl)-3-ferrocenyl-6,7-dimethoxy-13,13-dimethyl-3H,13H-indeno[2',3':3,4]naphtho[1,2-b]pyran, 3-(4-Fluorophenyl)-3-ferrocenyl-6,7-dimethoxy-13-ethyl-13-hydroxy-3H,13H-indeno[2',3':3,4]naphtho[1 ,2-b]pyran, 3-(4-Fluorophenyl)-3-ferrocenyl-6-morpholino-7-methoxy-13-ethyl-13-hydroxy-3H,13H-indeno[2',3':3,4]naphtho[1 ,2-b]pyran, 3-(4-Fluorophenyl)-3-phenyl-13,13-dimethyl-3H,13H-indeno[2',3':3,4]naphtho[1,2-b]pyran, 3-(4-Methoxyphenyl)-3-(4-morpholinophenyl)-6,7-dimethoxy-13-ethyl-13-methoxy-3H,13H-indeno[2,1-f]naphtho[1,2-b]pyran, 3-(4-Methoxyphenyl)-3-(2,3-dihydrobenzofur-5-yl)-13-acetoxy-6,11-dimethoxy-13-methyl-indeno[2,1-f]naphtho[1,2-b]pyran, 3-(4-Methoxyphenyl)-3-(2,3-dihydrobenzofur-5-yl)-13-hydroxy-13-methyl-indeno[2, 1-f]naphtho[1 ,2-b]pyran, 3-(4-Methoxyphenyl)-3-(3,4-dirnethoxyphenyl)-6, 11-dimethyl-13,13-dipropyl-indeno[2,1-f]naphth0[1,2-b]pyran, 3-(4-Methoxyphenyl)-3-(3-methyl-4-methoxyphenyl)-13-hydroxy-indeno[2,1-f]naphtho[1,2-b]pyran, 3-(4-Methoxyphenyl)-3-(4-dimethylaminophenyl)-6,7-dimethoxy-13,13-dimethyl-3H,13H-indeno[2,1-f]naphtho[1,2-b]pyran, 3-(4-Methoxyphenyl)-3-(4-morpholinophenyl)-16-(ethoxycarbonyl)methyl-16-hydroxy-3,16-di[H]-benzofuro[2',3':7 ,8]indeno[2',3':3,4]naphtho[1.2-b]pyran, 3-(4-Methoxyphenyl)-3-(4-morpholinophenyl)-6,11-difluoro-13,13-dimethyl-3H,13H-indeno[2',3':3,4]naphtho[1,2-b]pyran, 3-(4-Methoxyphenyl)-3-(4-morpholinophenyl)-6,7,10,11-tetramethoxy-13-hydroxy-13-butyl-3H,13H-indeno[2,1-f]naphtho[1,2-b]pyran, 3-(4-Methoxyphenyl)-3-(4-morpholinophenyl)-6,7,10,11-tetramethoxy-13-hydroxy-13-ethyl-3H,13H-indeno[2,1-f]naphtho[1,2-b]pyran, 3-(4-Methoxyphenyl)-3-(4-morpholinophenyl)-6,7-dimethoxy-13,13-dimethyl-3H,13H-indeno[2,1-f]naphtho[1,2-b]pyran, 3-(4-Methoxyphenyl)-3-(4-morpholinophenyl)-6,7-dimethoxy-13-hydroxy-13-ethyl-3H,13H-indeno[2,1-f]naphtho[1,2-b]pyran, 3-(4-Methoxyphenyl)-3-(4-morpholinophenyl)-6,7-dimethoxy-13-hydroxy-13-methyl-3H,13H-indeno[2,1-f]naphtho[1,2-b]pyran, 3-(4-Methoxyphenyl)-3-(4-morpholinophenyl)-6,7-dimethoxy-13-methoxy-13-methyl-3H,13H-indeno[2,1-f]naphtho[1,2-b]pyran, 3-(4-Methoxyphenyl)-3-(4-morpholinophenyl)-6,7-dimethoxy-13-phenyl-3H,13H-indeno[2,1-f]naphtho[1 ,2-b]pyran, 3-(4-Methoxyphenyl)-3-(5-methylthiophen-2-yl)-6,11-dichloro-13,13-dimethyl-3H,13H-indeno[2',3':3,4]naphtho[1,2-b]pyran, 3-(4-Methoxyphenyl)-3,9-diphenyl-3H-9H-indeno[3',2':3,4]naphtho[1,2-b]pyran, 3-(4-Methoxyphenyl)-3-phenyl-6-bromo-13,13-dimethyl-3H,13H-indeno[2',3':3,4]naphtho[1,2-b]pyran, 3-(4-Methoxyphenyl)-3-phenyl-9-(3-methoxyphenyl)-11-methoxy-3H-9H-indeno[3',2':3,4]naphtho[1,2-b]pyran, 3-(4-Methoxyphenyl)-3-phenyl-9,9-dimethyl-11-methoxy-3H-9H-indeno[3',2':3,4]naphtho[1,2-b]pyran, 3-(4-Methoxyphenyl)-3-phenyl-9,9-dimethyl-3H-9H-benzo[4",5"]indeno[3',2':3,4]naphtho[1,2-b]pyran, 3-(4-Methoxyphenyl)-3-phenyl-9,9-dimethyl-7,11-dimethoxy-3H-9H-indeno[3',2':3,4]naptho[1,2-b]pyran, 3-(4-Methoxyphenyl)-3-phenyl-9-methyl-11,13-dimethoxy-3H-9H-indeno[3',2':3,4]naphtho[1,2-b]pyran, 3-(4-Methoxyphenyl)-3-phenyl-9-methyl-11-methoxy-9-(3-methoxyphenyl)-3H-9H-indeno[3',2':3,4]naphtho[1,2-b]pyran, 3-(4-Methylphenyl)-3-(4-morpholinophenyl)-6,11-difluoro-13,13-dimethyl-3H,13H-indeno[2',3':3,4]naphtho[1,2-b]pyran, 3-(4-Morpholinophenyl)-3,9-diphenyl-3H-9H-indeno[3',2':3,4]naphtho[1,2-b]pyran, 3-(4-Morpholinophenyl)-3-phenyl-13,13-dimethyl-3H,13H-indeno[2',3':3,4]naphtho[1,2-b]pyran, 3-(4-Morpholinophenyl)-3-phenyl-6,11-difluoro-13,13-dimethyl-3H,13H-indeno[2',3':3,4]naphtho[1,2-b]pyran, 3-(4-Morpholinophenyl)-3-phenyl-6,7-dimethoxy-13-hydroxy-13-butyl-3H,13H-indeno[2,1-f]naphtho[1,2-b]pyran, 3-(4-Morpholinophenyl)-3-phenyl-6,7-dimethoxy-13-hydroxy-13-ethyl-3H,13H-indeno[2,1-f]naphtho[1,2-b]pyran, 3-(4-Morpholinophenyl)-3-phenyl-6-bromo-13,13-dimethyl-3H,13H-indeno[2',3':3,4]naphtho[1,2-b]pyran, 3-(4-Morpholinophenyl)-3-phenyl-9 ,9-dimethyl-7, Ildimethoxy-3H-9H-indeno[3',2':3,4]naphtho[1,2-b]pyran, 3,3,9-Triphenyl-3H-9H-indeno[3',2':3,4]naphtho[1,2-b]pyran, 3,3-Di(4-methoxyphenyl)-6, 11-dimethyl-13-(1-methylethyl)-13-hydroxy-indeno[2,1-f]naphtho[1,2-b]pyran, 3,3-Di(4-fluorophenyl)-6,11-di(methoxycarbonyl)-13,13-dimethyl-3H,13H-indeno[2',3':3,4]naphtho[1,2-b]pyran, 3,3-Di(4-Fluorophenyl)-6,11-dichloro-13,13-dimethyl-3H,13H-indeno[2',3':3,4]naphtho[1,2-b]pyran, 3,3-Di(4-fluorophenyl)-6,11-dicyano-13,13-dimethyl-3H,13H-indeno[2',3':3,4]naphtho[1,2-b]pyran, 3,3-Di(4-fluorophenyl)-6,11-difluoro-13,13-dimethyl-3H,13H-indeno[2',3':3,4]naphtho[1,2-b]pyran, 3,3-Di(4-Fluorophenyl)-6-cyano-13,13-dimethyl-3H,13H-indeno[2',3':3,4]naphtho[1,2-b]pyran, 3,3-Di(4-fluorophenyl)-6-methoxycarbonyl-13,13-dimethyl-3H,13H-indeno[2',3':3,4]naphtho[1,2-b]pyran, 3,3-Di(4-methoxyphenyl)-13-hydroxy-13-methyl-indeno[2,1-f]naphtho[1,2-b]pyran, 3,3-Di(4-methoxyphenyl)-16-(ethoxycarbonyl)methyl-16-hydroxy-3,16-di[H]benzofuro[2',3':7,8]indeno[2',3':3,4]naphtho[1,2-b)pyran, 3,3-Di(4-methoxyphenyl)-16-hydroxy-16-ethyl-16H-benzofuro[2' ,3 ':7 ,8]indeno[2' ,3' :3,4]naphtho[1,2-b]pyran, 3,3-Di(4-methoxyphenyl)-16-hydroxy-16-ethyl-16H-benzofuro[2",3":6',7']indeno[3',2':4,3]naphtho[1,2-b]pyran, 3,3-Di(4-methoxyphenyl)-16-hydroxy-16H-benzofuro[2",3":6',7']indeno[3',2':4,3]naphtho[1,2-b]pyran, 3,3-Di(4-methoxyphenyl)-6, 11-dimethyl-13-butyl-13-hydroxy-indeno[2,1-f]naphtho[1,2-b]pyran, 3,3-Di(4-methoxyphenyl)-6,11-di(methoxycarbonyl)-13,13-dimethyl-3H,13H-indeno[2',3':3,4]naphtho[1,2-b]pyran, 3,3-Di(4-methoxyphenyl)-6,11-dichloro-13,13-dimethyl-3H,13H-indeno[2',3':3,4]naphtho[1,2-b]pyran, 3,3-Di(4-methoxyphenyl)-6,11-difluoro-13,13-dimethyl-3H,13H-indeno[2',3':3,4]naphtho[1,2-b]pyran, 3,3-Di(4-methoxyphenyl)-6,11-dirnethyl-13-methoxyindeno[2,1-f]naphtho[1,2-b]pyran, 3,3-Di(4-methoxyphenyl)-6,7,10,11-tetramethoxy-13,13-diethyl-3H,13H-indeno[2,1-f]naphtho[1,2-b]pyran, 3,3-Di(4-methoxyphenyl)-6,7,10,11-tetramethoxy-13,13-dimethyl-3H,13H-indeno[2,1-f]naphtho[1,2-b]pyran, 3,3-Di(4-methoxyphenyl)-6,7,10,11-tetramethoxy-13-hydroxy-13-ethyl-3H,13H-indeno[2,1-f]naphtho[1 ,2-b]pyran, 3,3-Di(4-methoxyphenyl)-6,7,8-trimethoxy-13-phenyl-3H,13H-indeno[2,1-f]naphtho[1,2-b]pyran, 3,3-Di(4-methoxyphenyl)-6,7-dimethoxy-13,13-dimethyl-3H,13H-indeno[2,1-f]naphtho[1,2-b]pyran, 3,3-Di(4-methoxyphenyl)-6,7-dimethoxy-13-hydroxy-13-butyl-3H,13H-indeno[2,1-f]naphtho[1 ,2-b]pyran, 3,3-Di(4-methoxyphenyl)-6,7-dimethoxy-13-hydroxy-13-ethyl-3H,13H-indeno[2,1-f]naphtho[1,2-b]pyran, 3,3-Di(4-methoxyphenyl)-6,7-dimethoxy-13-phenyl-3H,13H-indeno[2,1-f]naphtho[1,2-b]pyran, 3,3-Di(4-methoxyphenyl)-6-methoxy-7-(3-(2-methacryloxyethyl)carbamyloxymethylenepiperidin-1-yl)-13,13-dimethyl-3H,13H-indeno[2',3':3,4]naphtho[1,2-b]pyran, 3,3-Di(4-methoxyphenyl)-6-methyl-11-fluoro-13,13-diethoxy-indeno[2,1-f]naphtho[1,2-b]pyran, 3,3-Di(4-methoxyphenyl)-9-(3-methoxyphenyl)-11-methoxy-3H-9H-indeno[3',2': 3,4]naphtho[1,2-b]pyran, 3,3-Di(4-methoxyphenyl)-9,9-dimethyl-11-fluoro-3H-9H-indeno[3',2':3,4]naphtho[1,2-b]pyran, 3,3-Di(4-methoxyphenyl)-9,9-dimethyl-11-methoxy-3H-9H-indeno[3',2':3,4]naphtho[1,2-b]pyran, 3,3-Di(4-methoxyphenyl)-9,9-dimethyl-7,11-dimethoxy-3H-9H-indeno[3',2':3,4]naphtho[1,2-b]pyran, 3,3-Di(4-methoxyphenyl)-9-methyl-11,13-dimethoxy-3H-9H-indeno[3',2':3,4]naphtho[1,2-b]pyran, 3,3-Di(4-methoxyphenyl)-9-methyl-11-methoxy-3H-9H-indeno[3',2':3,4]naphtho[1,2-b]pyran, 3,3-Di(4-methoxyphenyl)-9-methyl-11 -methoxy-9-(3-methoxyphenyl)-3H-9H-indeno[3',2':3,4]naphtho[1,2-b]pyran, 3,3-Di(4-methoxyphenyl)-9-phenyl-3H-9H-indeno[3',2':3,4]naphtho[1,2-b]pyran, 3,3-Diphenyl-13,13-dimethyl-3H,13H-indeno[2',3':3,4]naphtho[1,2-b]pyran, 3,3-Diphenyl-6,11-dicyano-13,13-dimethyl-3H,13H-indeno[2',3':3,4]naphtho[1,2-b]pyran, 3-Hexyl-7,7-diphenyl-2,4-dioxo-2,3,4,7-tetrahydro[1,3]oxazino[5',6':3,4]naphtho[1,2-b]pyran, 3-Methyl-7,7-diphenyl-2,4-dioxo-2,3,4,7-tetrahydro[1,3]oxazino[5',6':3,4]naphtho[1,2-b]pyran, 3-Phenyl-3-(4-(2-(2-(2-(2-(2-(2-(2-(2-methacryloxyethoxy)ethoxy)ethoxy)ethoxy)ethoxy)ethoxy)carbonylethyl)carboxyethoxy)phenyl)-6-methoxy-7-morpholino-13,13-dimethyl-3H,13H-indeno[2',3':3,4]naphtho[1,2-b]pyran, 3-Phenyl-3-(4-(2-(2-methacryloxyethyl)carbamyloxyethoxy)phenyl)-6,7-dimethoxy-13,13-dimethyl-3H,13H-indeno[2',3':3,4]naphtho[1,2-b]pyran, 3-Phenyl-3-(4-(2-(2-methacryloxyethyl)carbamyloxyethoxy)phenyl)-6-methoxy-7-piperidino-13,13-dimethyl-3H,13H-indeno[2',3':3,4]naphtho[1,2-b]pyran, 3-Phenyl-3-(4-(2-methacryloxyethyl)carbamyloxyphenyl)-6,7-dimethoxy-13,13-dimethyl-3H,13H-indeno[2',3':3,4]naphtho[1,2-b]pyran, 3-Phenyl-3-(4-(4-(2-(2-methacryloxyethyl)carbanlyloxyethyl)piperazin-1-yl)phenyl)-13,13-dimethyl-3H,13H-indeno[2',3':3,4]naphtho[1,2-b]pyran, 3-Phenyl-3-(4-(4-(2-methacryloxyethyl)carbamylpiperazin-1-yl)phenyl)-6,11-dimethoxy-13,13-dimethyl-3H,13H-indeno[2',3':3,4]naphtho[1,2-b]pyran, 3-Phenyl-3-(4-(4-phenylpiperazino)phenyl)-6-methoxy-7-(4-(2-methacryloxyethyl)carbamyloxypiperidin-1-yl)-13,13-dimethyl-3H,13H-indeno[2',3':3,4]naphtho[1,2-b]pyran, 3-Phenyl-3-(4-methoxyphenyl)-16-(ethoxycarbonyl)methyl-16-hydroxy-3.16-di[H)-benzofuro[2",3":6',7']indeno[3',2':4,3]naphtho[1,2-b]pyran, 3-Phenyl-3-(4-methoxyphenyl)-6-methoxy-7-(3-(2-(2-(2-(2-(2-(2-(2-methacryloxyethoxy)ethoxy)ethoxy)ethoxy)ethoxy)ethoxy)carbonylethyl)carboxymethylenepiperidin-1-yl)-13,13-dimethyl-3H, 13H-indeno [2',3':3,4]naphtho[1,2-b]pyran, 3-Phenyl-3-(4-methoxyphenyl)-6-methoxy-7-(4-(2-(2-(2-(2-(2-(2-(2-methacryloxyethoxy)ethoxy)ethoxy)ethoxy)ethoxy)ethoxy)carbonylethyl)carboxypiperidin-1-yl)-13,13-dimethyl-3H,13H-indeno[2',3':3,4]naphtho[1,2-b]pyran, 3-Phenyl-3-(4-methoxyphenyl)-6-methoxy-7-(4-(2-methacryloxyethyl)carbamylpiperazin-1-yl)-13,13-dimethyl-3H,13H-indeno[2',3':3,4]naphtho[1,2-b]pyran, 3-Phenyl-3-(4-morpholinophenyl)-16-(ethoxycarbonyl)methyl-16-hydroxy-3,16-di[H]benzofuro[2",3":6',7']indeno[3',2':4,3]naphtho[1.2-b]pyran, 3-Phenyl-3-(4-morpholinophenyl)-6,7,10,11-tetramethoxy-13,13-dimethyl-3H,13H-indeno[2,1-f]naphtho[1,2-b]pyran, 3-Phenyl-3-(4-morpholinophenyl)-6-methoxy-7-(3-(2-(2-(2-(2-(2-(2-(2-methacryloxyethoxy)ethoxy)ethoxy)ethoxy)ethoxy)ethoxy)carbonylethyl)carboxymethylenepiperidin-1-yl)-13,13-dimethyl-3H,13H-indeno[2',3':3,4]naphtho[1,2-b]pyran, 3-Phenyl-3-(4-morpholinophenyl)-6-methoxy-7-(3-(2-methacryloxyethyl)carbamyloxymethylenepiperidin-1-yl)-13,13-dimethyl-3H,13H-indeno[2',3':3,4]naphtho[1,2-b]pyran, 3-Phenyl-3-(4-morpholinophenyl)-6-methoxy-7-(4-(2-(2-(2-(2-(2-(2-(2-methacryloxyethoxy)ethoxy)ethoxy)ethoxy)ethoxy)ethoxy)carbonylethyl)carboxypiperidin-1-yl)-13,13-dimethyl-3H,13H-indeno[2',3':3,4]naphtho[1,2-b]pyran, 3-Phenyl-3-(4-morpholinophenyl)-6-methoxy-7-(4-(2-methacryloxyethyl)carbamyloxypiperidin-1-yl)-13,13-dimethyl-3H,13H-indeno[2',3':3,4]naphtho[1,2-b]pyran, 3-Phenyl-3-(4-morpholinophenyl)-6-methoxy-7-(4-(2-methacryloxyethyl)carbamylpiperazin-1-yl)-13,13-dimethyl-3H,13H-indeno[2',3':3,4]naphtho[1,2-b]pyran, 3-Phenyl-3-(4-piperidinophenyl)-13,13-dimethyl-3H,13H-indeno[2',3':3,4]naphtho[1,2-b]pyran, 3-Phenyl-3-(4-piperidinophenyl)-6,11-dichloro-13,13-dimethyl-3H,13H-indeno[2',3':3,4]naphtho[1,2-b]pyran, 3-Phenyl-3-(4-piperidinophenyl)-6,11-difluoro-13,13-dimethyl-3H,13H-indeno[2',3':3,4]naphtho[1,2-b]pyran, 3-Phenyl-3-(4-piperidinophenyl)-6,11-difluoro-13,13-dimethyl-3H,13H-indeno[2',3':3,4]naphtho[1,2-b]pyran, 3-Phenyl-3-ferrocenyl-13,13-dimethyl-3H,13H-indeno[2',3':3,4]naphtho[1,2-b]pyran, 5-Ethoxy-2-(2-fluorophenyl)-7-methoxy-2-(4-methoxyphenyl)-2H-pyrano[3,2-c]quinolin, 5-Ethoxy-2-(2-fluorophenyl)-9-methoxy-2-(4-methoxyphenyl)-2H-pyrano[3,2-c]quinolin, 5-Ethoxy-2,2-diphenyl-2H-pyrano[3,2-c]quinolin, 5-Ethoxy-3-(2-fluorophenyl)-3-(4-methoxy)-2H-[1,3]-dioxolo[4,5-g]pyrano[3,2-c]quinolin, 5-Ethoxy-7,9-dimethoxy-2,2-diphenyl-2H-pyrano[3,2-c]quinolin, 5-Ethoxy-7-methoxy-2,2-diphenyl-2H-pyrano[3,2-c]quinolin, 5-Ethoxy-9-fluoro-2,2-diphenyl-2H-pyrano[3,2-c]quinolin, 5-Ethoxy-9-methoxy-2,2-diphenyl-2H-pyrano[3,2-c]quinolin, 5-Methoxy-2,2-diphenyl-2H-pyrano[3,2-c]quinolin, 6-(4-Methoxyphenyl)-8,9-dimethoxy-3-(2-fluorophenyl)-3-(4-methoxyphenyl)-3H-naphtho[2,1-b]pyran, 6-(4-Methoxyphenyl)-8,9-dimethoxy-3,3-diphenyl-3H-naphtho[2,1-b]pyran, 6-(4-Methylphenyl)-3-(2-fluorophenyl)-3-(4-methoxyphenyl)-3H-naphtho[2,1-b]pyran, 6-(4-Methylphenyl)-3,3-diphenyl-3H-naphtho[2,1-b]pyran, 6-Phenyl-8,9-dimethoxy-3-(2-fluorophenyl)-3-(4-methoxyphenyl)-3H-naphtho[2,1-b]pyran, 6-Phenyl-8,9-dimethoxy-3,3-diphenyl-3H-naphtho[2,1-b]pyran, 7-(4-Methoxyphenyl)-7-phenyl-2-pentyl-4-oxo-4H-7H-[1,3]dioxino[5',4':3,4]naphtho[1,2-b]pyran, 7,7-Di(4-methoxyphenyl)-4-oxo-4H-7H-[1,3]dioxino[5',4':3,4]naphtho[1,2-b]pyran, 7,7-Diphenyl-1,2,4,7-tetrahydro-2,2-dimethylpyrano[3',4':3,4]naphtho[1,2-b]pyran, 7,7-Diphenyl-2-(1-phenylethyl)-4-oxo-4H-7H-[1,3]dioxino[5',4':3,4]naphtho[1,2-b]pyran, 7,7-Diphenyl-2-(2-methylpropyl)-4-oxo-4H -7H-[1,3]dioxino[5',4':3,4]naphtho[1,2-b]pyran, 7,7-Diphenyl-2-ethyl-4-oxo-4H-7H-[1,3]dioxino[5',4':3,4]naphtho[1,2-b]pyran, 7,7-Diphenyl-2-pentyl-4-oxo-4H-7H-[1,3]dioxino[5',4':3,4]naphtho[1,2-b]pyran und 7,7-Diphenyl-4-oxo-4H-7H-[1,3]dioxino[5',4':3,4]naphtho[1,2-b]pyran, 2,2-Bis(4-methoxyphenyl)-5-(N-methylpyrrol-2-yl)-6-acetoxy-2H-naphtho[1.2-b]pyran, 2,2-Bis(4-methoxyphenyl)-5-(2,4-dimethoxyphenyl)-6-acetoxy-2H-naphtho[1,2-b]pyran, 2,2-Bis(4-methoxyphenyl)-5-phenylthio-6-hydroxy-2H-naphtho[1,2-b]pyran, 2,2-Bis(4-methoxyphenyl)-5-phenylthio-6-acetoxy-2H-naphtho[1,2-b]pyran, 2,2-Bis(4-methoxyphenyl)-5-chloro-6-acetoxy-2H-naphtho[1,2-b]pyran, 2,2-Bis(4-methoxyphenyl)-5-methyl-6-acetoxy-2H-naphtho[1,2-b]pyran, 2,2'-Spiroadamantylen-5-phenylthio-6-hydroxy-2H-naphtho[1,2-b]pyran, 2,2'-Spiroadarnantylen-5-phenylthio-6-acetoxy-2H-naphtho[1,2-b]pyran, 2,2'-Spiroadamantylen-5-methyl-6-methoxy-2H-naphtho[1,2-b]pyran, 2,2-Bis(4-methoxyphenyl)-5-diphenylmethylol-6-hydroxy-2H-naphtho[1,2-b]pyran, 2,2-Bis(4-methoxyphenyl)-5-diphenylmethylol-6-methoxy-2H-naphtho[1,2-b]pyran, 1 ,3-Trimethylspiro[indolin-2,2'-[2H]-naphtho[1,2-b]pyran], 1,3,3,5,6-Pentamethylspiro[indolin-2,2'-[2H]-naphtho[1,2-b]pyran], 1,3-Trimethyl-5-methoxyspiro[indolin-2,2'-[2H]-naphtho[1,2-b]pyran], 1,3-Trimethyl-6'-chlorospiro[indolin-2,2' -[2H]-naphthol[1,2-b]pyran] und 1,3,3-Trimethyl-6'-nitrospiro[indolin-2,2'-[2H]-naphthol[1,2-b]pyran] und Mischungen davon.

Die Fulgid- und die Fulgimidverbindungen können insbesondere ausgewählt sein aus der Gruppe aufweisend oder bestehend aus (E)-α-(1,2,5-trimethyl-3-pyrryl)ethyliden(isopropyliden)succinicanhydrid, (E)-α-(2,5-dimethyl-1-phenyl-3-pyrryl)ethyliden(isopropyliden)succinicanhydrid, (E)-α-(2,5-dimethyl-1-p-tolyl-3-pyrrl)ethylidene(isopropyliden)succinicanhydrid, (E)-α-(1,5-diphenyl-2-methyl-3-pyrryl)ethyliden(iso[propylidene)succinicanhydrid und (E)-α-(2,5-dimethyl-1-phenyl-3-pyrryl)ethyliden(dicyclopropylmethylen)succinicanhydrid und Mischungen davon.

Die Diarylethenverbindungen können insbesondere ausgewählt sein aus der Gruppe aufweisend oder bestehend aus 1,2-Bis(2-(2-benzothiazolyi)-benzo[b]thien-3-yl)perfluorocyclopenten und 1,2-Bis-(2,5-bis-(2-benzothiazolyl)-thien3-yl)perfluorocyclopenten und Mischungen davon.Die Oxazinverbindungen können ausgewählt sein aus der Gruppe aufweisend Benzoxazinverbindungen, Naphthoxazinverbindungen, Spiro-oxazinverbindungen, Spiro(indolin)naphthoxazinverbindungen, Spiro(indolin)pyridobenzoxazinverbindungen, Spiro(benzindolin)pyridobenzoxazinverbindungen, Spiro(benzindolin)naphthoxazinverbindungen, Spiro(indolin)benzoxazinverbindungen, Spiro(indolin)fluoranthenoxazinverbindungen, Spiro(indolin)quinoxazinverbindungen und Mischungen davon.

Bei den dimeren Lactonverbindungen kann es sich beispielsweise um Dibenzofuranon handeln.

Bei den dimeren Imidazolverbindungen kann es sich beispielsweise um 2,4,5-Triarylimidazol handeln.

Bei den Bicycloverbindungen kann es sich insbesondere um Bicyclo[4.2.0]octan (BCO) oder Bicyclo[4.2.0]octanverbindungen (BCO-Verbindungen) handeln.

In einer bevorzugten Ausführungsform hat das Mechanophor die nachstehende Formel I: 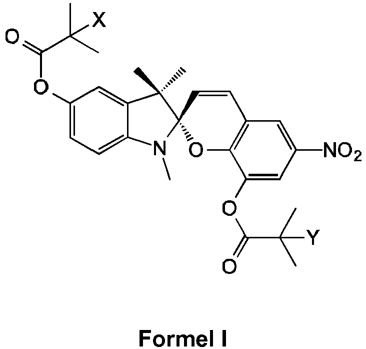 wobei X und Y gleich oder verschieden sein und unabhängig voneinander eine Alkylgruppe, insbesondere Methylgruppe (CH₃), ein Halogenatom, vorzugsweise Bromatom (Br) oder Chloratom (Cl), oder ein Wasserstoff (H) bedeuten können.

Das Mechanophor gemäß Formel I ist bei Belastung in eine azyklische Verbindung gemäß Formel II überführbar: 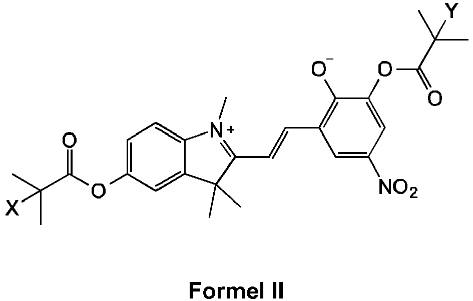 wobei X und Y gleich oder verschieden sein und unabhängig voneinander eine Alkylgruppe, insbesondere Methylgruppe (CH₃), ein Halogenatom, vorzugsweise Bromatom (Br) oder Chloratom (Cl), oder ein Wasserstoff (H) bedeuten können.

Das Mechanophor gemäß Formel I ist farblos oder gelblich, wohingegen das Mechanophor gemäß Formel II eine rote oder violette Färbung besitzt und stark fluoreszierend ist. Auf diese Weise können auf das erfindungsgemäße Medizinprodukt einwirkende Kräfte visualisiert werden, insbesondere mittels Fluoreszenzmessungen.

In einer weiteren Ausführungsform weist das Medizinprodukt Additive auf. Die Additive können ausgewählt sein aus der Gruppe aufweisend oder bestehend aus antiproliferative Wirkstoffe, antimikrobielle, insbesondere antibiotische, Wirkstoffe, entzündungshemmende Wirkstoffe, schmerzlindernde Wirkstoffe, geruchsbekämpfende Wirkstoffe, desinfizierende Wirkstoffe, biologische Wachstumsfaktoren, röntgenopake Stoffe und Mischungen davon.

Bei dem Medizinprodukt handelt es sich um ein Implantat oder Implantatteil, insbesondere humanmedizinisches oder -chirurgisches Implantat oder Implantatteil, wobei es sich dabei um eine Gelenkprothese, ein Teil einer Gelenkprothese, ein Probeimplantat oder ein Probeimplantatteil handelt.

Das Implantat oder Implantatteil kann grundsätzlich für einen dauerhaften oder nur vorübergehenden Verbleib im Körper eines menschlichen oder tierischen Patienten vorgesehen sein. Entsprechend kann es sich bei dem Implantat oder Implantatteil um ein permanentes oder temporäres Implantat oder Implantatteil handeln. Beispielsweise kann das Implantat oder Implantatteil dazu vorgesehen sein, lediglich für die Dauer einer Infektsanierung in einem Situs zu verbleiben. Das Implantat oder Implantatteil ist bevorzugt zur Anwendung bei der Behandlung eines Gelenkdefekts, insbesondere Knie- oder Hüftgelenkdefekts, vorgesehen.

Bevorzugt ist das Implantat oder Implantatteil zur Durchführung eines arthroplastischen, insbesondere knie- oder hüftarthroplastischen, Eingriffes, vorgesehen.

In einer weiteren Ausführungsform ist das Implantat oder Implantatteil zur Anwendung bei der Messung und/oder Beurteilung der Band- und/oder Sehnenspannung im Körper eines menschlichen oder tierischen Patienten, bevorzugt zur Anwendung bei der Messung und/oder Beurteilung der Band- und/oder Sehnenspannung bei menschlichen oder tierischen Gelenken, vorgesehen.

Gemäß einer besonders bevorzugten Ausführungsform handelt es sich bei dem Implantat um eine Gelenkprothese.

Bei dem Implantatteil handelt es sich vorzugsweise um ein Teil einer Gelenkprothese, bevorzugt um einen Kopf oder Kopfteil, eine Pfanne oder ein Pfannenteil, einen Einsatz oder eine Einlage (Inlay), insbesondere Cup-Inlay, oder eine Gleitfläche. Mit anderen Worten handelt es sich bei Implantatteil vorzugsweise um einen Gelenkkopf oder Gelenkkopfteil, eine Gelenkpfanne oder ein Gelenkpfannenteil, einen Gelenkeinsatz oder eine Gelenkeinlage (Gelenk-Inlay) oder eine Gelenk-Gleitfläche.

Besonders bevorzugt ist das Implantatteil ausgewählt aus der Gruppe aufweisend oder bestehend aus Hüftgelenkskopf oder Hüftgelenkskopfteil, Hüftgelenkspfanne oder Hüftgelenkspfannenteil, Hüftgelenkseinsatz oder Hüftgelenkseinlage (Hüftgelenks-Inlay), Hüftgelenksgleitfläche, Kniegelenkskopf oder Kniegelenkskopfteil, Kniegelenkspfanne oder Kniegelenkspfannenteil, Kniegelenkseinsatz oder Kniegelenkseinlage (Kniegelenks-Inlay) und Kniegelenksgleitfläche. Die bereits erwähnte Gelenkprothese ist in einer weitergehenden Ausführungsform ausgewählt aus der Gruppe aufweisend Kniegelenkprothese, Hüftgelenkprothese, Knöchelgelenkprothese, Schultergelenkprothese, Kiefergelenkprothese, Ellenbogengelenkprothese, Fingergelenkprothese und Wirbelsäulengelenkflächenprothese.

In einer weiteren Ausführungsform (nicht erfindungsgemäß) handelt es sich beim dem Implantat um eine künstliche Tibia oder eines künstlichen Femur.

In einer weiteren Ausführungsform handelt es sich bei dem Implantat oder Implantatteil um ein Implantat für die Wirbelsäule, d. h. um ein sogenanntes Wirbelsäulenimplantat, vorzugsweise um ein ein Zwischenwirbelimplantat (nicht erfindungsgemäß) oder um ein Implantatteil für die Wirbelsäule, d. h. um ein sogenanntes Wirbelsäulenimplantatteil, vorzugsweise um ein Zwischenwirbelimplantatteil (nicht erfindungsgemäß). Ein Zwischenwirbelimplantat wird nach Entfernung einer Bandscheibe in Zwischenwirbelräume eingesetzt.

In einer weiteren Ausführungsform handelt es sich bei dem Implantat um ein Meniskusimplantat. Das Meniskusimplantat kann dazu gestaltet sein, einen Meniskus vollständig oder nur teilweise zu ersetzen. Vorzugsweise weist das Meniskusimplantat wenigstens eine Artikulationsfläche, insbesondere eine oder zwei Artikulationsflächen, zum Bewirken einer Artikulation mit einem Femur (Oberschenkelknochen) und wenigstens eine Artikulationsfläche, insbesondere eine oder zwei Artikulationsflächen, zum Bewirken einer Artikulation mit einer Tibia (Schienbein) auf.

In einer besonders bevorzugten Ausführungsform handelt es sich bei dem Implantat oder Implantatteil, um ein sogenanntes Probeimplantat oder Probeimplantatteil. Unter dem Ausdruck "Probeimplantat" soll im Sinne der vorliegenden Erfindung ein Implantat oder Instrument, vorzugsweise zur Verwendung im Rahmen einer chirurgischen Operation zum Gelenkersatz, zum Gelenkoberflächenersatz oder zum Bandscheibenersatz, verstanden werden. Das Probeimplantat ist in seiner Form einem finalen, vorzugsweise einem für den Ersatz eines Gelenks, einer Gelenkoberfläche oder einer Bandscheibe, vorgesehenen, Implantat, oder einem finalen medizinischen Instrument nachempfunden. Entsprechend soll unter dem Ausdruck "Probeimplantatteil" ein Teil eines Probeimplantats verstanden werden.

Mittels des Probeimplantats kann insbesondere der Sitz eines finalen Implantats und die damit mögliche Gelenkkinematik getestet werden.

Darüber hinaus kann das Probeimplantat zusätzliche Funktionen erfüllen. So kann das Probeimplantat beispielsweise als Aufnahme für weitere Instrumente oder als Führungsinstrument für weitere Instrumente dienen. Mittels eines erfindungsgemäßen Probeimplantats kann beispielsweise mit besonderem Vorteil der Pressfit kontrolliert werden, mit welchem zementfrei Implantate am/im Knochen verankert werden. In diesem Fall kann sich das Probeimplantat beispielsweise hin zu einer klar definierten Farbe verfärben. Wird diese Farbe nicht erreicht, ist dies ein Hinweis auf einen zu geringen Pressfit. Wird dagegen eine andere Farbe angezeigt, kann dies als Indiz für einen zu hohen Pressfit angesehen werden.

Weiterhin kann das Probeimplantat bei der Durchführung von Repositionsmanövern von Vorteil sein. So können mittels eines entsprechend ausgestalteten Probeimplantats für sämtliche Repositionsvorgänge über entsprechende Hebel, wie beispielsweise für die Wirbelrotation bei Deformitäten, eine Aussage über aufzubringende Kräfte oder Momente gemacht werden. Es kann beispielsweise die Steifigkeit der Wirbelsäule bewertet werden, und es können eventuell weitere Maßnahmen zum Release für ein verbessertes Korrekturergebnis durchgeführt werden. Hierdurch kann eine ungleichmäßige in vivo Belastung eines Implantats sowie eine ungleichmäßige Belastung von Körpergewebe, insbesondere von Weich- und/oder Knochengewebe, vermieden werden. Dies trägt in besonders vorteilhafter Weise zu einer langen in vivo Stand-/Überlebenszeit eines Implantats bei.

Beispielsweise kann das Probeimplantat oder Probeimplantatteil ausgewählt sein aus der Gruppe aufweisend oder bestehend aus Probegelenk, Probegelenkteil, Probegelenkkopf oder Probegelenkkopfteil, Probegelenkpfanne oder Probegelenkpfannenteil, Probegelenkeinsatz oder Probegelenkeinlage, insbesondere Probe-Cup-Inlay, Probehüftgelenk, Probehüftgelenkteil, Probehüftgelenkkopf oder Probehüftgelenkkopfteil, Probehüftgelenkpfanne oder Probehüftgelenkpfannenteil, Probegleitfläche, Probegelenkgleitfläche, Probetibia und Probefemur.

Verfärben sich beispielsweise die beiden Kompartimente einer kniearthroplastischen Probegleitfläche unterschiedlich, ist das ein Hinweis für den Anwender, vorzugsweise Arzt, dass die laterale und mediale Bandspannung noch unterschiedlich eingestellt sind. Durch die unterschiedlichen Farben oder Farbänderungen erhält er die Information, welche Seite straffer und welche Seite laxer ist. Im Fall eines Probefemurs kann die Position und Ausrichtung der Patella während der Beugung/Streckung und somit die korrekte Position der Patella in Relation zu einem Femurimplantat bestimmt werden.

In einer weiteren Ausführungsform handelt es sich bei dem Probeimplantat oder Probeimplantatteil um ein Probeimplantat für die Wirbelsäule, d. h. um ein sogenanntes Wirbelsäulenprobeimplantat, vorzugsweise um ein ein Zwischenwirbelprobeimplantat, oder um ein Probeimplantatteil für die Wirbelsäule, d. h. um ein sogenanntes Wirbelsäulenprobeimplantatteil, vorzugsweise um ein Zwischenwirbelprobeimplantatteil. Ein derartiges Probeimplantat oder Probeimplantatteil kann mit besonderem Vorteil nach dem Einsetzen Informationen zur Auflagefläche und insbesondere zu möglichen Belastungsspitzen liefern.

In einer weiteren Ausführungsform handelt es sich bei dem Probeimplantat um ein Meniskusprobeimplantat. Vorzugsweise weist das Meniskusprobeimplantat wenigstens eine Artikulationsfläche, insbesondere eine oder zwei Artikulationsflächen, zum Bewirken einer Artikulation mit einem Femur (Oberschenkelknochen) und wenigstens eine Artikulationsfläche, insbesondere eine oder zwei Artikulationsflächen, zum Bewirken einer Artikulation mit einer Tibia (Schienbein) auf. Bevorzugt weisen die wenigstens eine Artikulationsfläche zum Bewirken einer Artikulation mit einem Femur und/oder die wenigstens eine Artikulationsfläche zum Bewirken einer Artikulation mit einer Tibia ein Mechanophor auf oder sind mit einer Beschichtung versehen, welche ein Mechanophor enthält. Dadurch ist insbesondere eine Beurteilung möglich, ob die in vivo auf die Artikulationsflächen einwirkenden Kräfte gleich groß sind.

In einer alternativen Ausführungsform (nicht erfindungsgemäß) handelt es sich bei dem Medizinprodukt um ein medizinisches, insbesondere chirurgisches, Instrument oder Instrumententeil, vorzugsweise um ein humanmedizinisches, insbesondere humanchirurgisches, Instrument oder Instrumententeil.

Bei dem Instrument oder Instrumententeil kann es sich insbesondere um ein Einmalinstrument (sogenanntes "single-use" Instrument) oder Einmalinstrumententeil handeln.

Bei dem Instrument oder Instrumententeil kann es sich insbesondere um ein Instrument oder Instrumententeil zur Kraftindikation, insbesondere zur Drehmoment- oder Spreizkraftindikation, handeln Beispielsweise kann es sich bei dem Instrument um einen Drehmomentschlüssel handeln. Das Erreichen eines geforderten Drehmoments bzw. das Überschreiten desselben kann beispielsweise mit besonderem Vorteil durch eine unterschiedliche Färbung des Mechanophors sichtbar gemacht werden.

In einer weitergehenden Ausführungsform handelt es sich bei dem Instrument um einen Spreizer.

In einer weiteren Ausführungsform handelt es sich bei dem Instrument um ein Instrument, insbesondere Spreizer, mit einem Festkörpergelenk. Unter dem Ausdruck "Festkörpergelenk" soll im Sinne der vorliegenden Erfindung ein Bereich eines medizinischen, insbesondere chirurgischen, Instruments verstanden werden, welcher eine Relativbewegung zwischen zwei Starrkörperbereichen des Instruments durch Biegung erlaubt. Das Festkörpergelenk ist dafür konzipiert, die Funktion eines konventionellen Gelenks bzw. Lagers zu übernehmen. Bevorzugt ist das Festkörpergelenk als ebenes Drehgelenk ausgestaltet. Ein derartiges Gelenk kann ein konventionelles Drehlager ersetzen und eine bestimmte Schwenkung zwischen zwei steifen Bereichen des Instruments ermöglichen. Das Festkörpergelenk kann beispielsweise als Blattfedergelenk, Kerbgelenk, Dreieckgelenk, Wagenradgelenk, Parallelgelenk oder Kreuzfedergelenk ausgestaltet sein.

Bevorzugt weist das Festkörpergelenk, insbesondere nur das Festkörpergelenk, das Mechanophor auf. Auf diese Weise kann mit besonderem Vorteil eine Art "Kraftanzeige" realisiert werden. Dies kann beispielsweise bei der Ermittlung von Gelenkspalten im Rahmen einer knieendoprothetischen Versorgung zur Anwendung kommen, um bei der Aufspreizung der Streck- und Beugespalte im medialen und lateralen Kompartement des Gelenks immer die gleiche Kraft aufzubringen. Auf diese Weise können gleichmäßige und insbesondere parallele Spalte erreicht werden, was wiederum für eine lange Standzeit der Implantate vorteilhaft ist. Die in diesem Abschnitt beschriebene Ausführungsform kann ferner bei Wirbelsäulenoperationen von Vorteil sein. Bei derartigen Operationen ist ebenfalls eine Aufspreizung erforderlich, nämlich eine Aufspreizung des Zwischenwirbelbereichs, um die korrekte Implantatgröße zu bestimmen und das Implantat zu applizieren. Ein weiterer Vorteil eines Spreizers bzw. Instruments mit einem Festkörpergelenk besteht in der Möglichkeit zur Warnung von Ermüdungsbrüchen. Festkörpergelenke, welche das Mechanophor aufweisen oder aus einer solchen Verbindung bestehen, können zudem kostengünstiger hergestellt werden und ohne weiteres konventionelle Gelenke in chirurgischen Instrumenten ersetzen. Dies bewirkt insgesamt eine kostengünstigere Herstellung von Instrumenten mit Festkörpergelenken. Dieser kommt bei der Herstellung von Einmalinstrumenten besonders stark zur Geltung. Ein weiterer Vorteil eines Festkörpergelenks mit dem erfindungsgemäß vorgesehenen Mechanophor besteht in der Möglichkeit, eine unerlaubt erfolgte Sterilisation anzuzeigen, um auf diese Weise die Wiederverwendung von Einmalinstrumenten zu verhindern.

In einer weiteren Ausführungsform (nicht erfindungsgemäß) handelt es sich bei dem Medizinprodukt um ein Festkörpergelenk für ein medizinisches, insbesondere chirurgisches, Instrument, vorzugsweise für ein humanmedizinisches, insbesondere humanchirurgisches, Instrument. Bezüglich weiterer Merkmale und Vorteile dieser Ausführungsform wird auf die in den vorhergehenden Absätzen gemachten Ausführungen Bezug genommen.

In einer weiteren Ausführungsform handelt es sich bei dem Instrument um ein Navigationsinstrument. Hierbei handelt es sich in der Regel um ein längliches Instrument. Mit besonderem Vorteil können an Navigationsinstrumenten auftretende Verformungen durch die Anwesenheit des Mechanophors erfasst werden und ggf. beim Tracking (bei der Nachführung) korrigiert werden.

In einer weiteren Ausführungsform ist das Instrument ein Retraktor. Unter dem Ausdruck "Retraktor" soll im Sinne der vorliegenden Erfindung ein chirurgisches Instrument verstanden werden, mit dessen Hilfe der Zugang zu einem Operationsfeld offengehalten oder auch erst ermöglicht wird. Bei dem Retraktor kann es sich beispielsweise um einen Retraktor für die Wirbelsäule, insbesondere für die lumbale Wirbelsäule, oder um einen Retraktor für die Hüftendoprothetik (sogenannter Hüftrektraktor) handeln.

Der Retraktor weist vorzugsweise einen Rahmen, zwei Retraktorarme sowie zwei Retraktorblätter auf. Der Rahmen ist vorzugsweise in Form einer geradlinigen Zahnstange ausgestaltet. Vorzugsweise ist einer der Retraktorarme mit einem Ende des Rahmens verbunden, insbesondere verschiebefest verbunden. Der andere Retraktorarm ist vorzugsweise längs des Rahmens verschiebbar gelagert, bevorzugt über eine Führungshülse. Die beiden Retraktorarme stehen jeweils bevorzugt senkrecht von dem Rahmen ab. Mit anderen Worten laufen die Retraktorarme bevorzugt parallel zueinander. Zweckmäßigerweise haben die beiden Retraktorarme dieselbe Länge. Die beiden Retraktorarme können jeweils weiterhin in Abschnitte, insbesondere zwei Abschnitte, untergliedert sein, welche über ein Gelenk miteinander verbunden sind. Bevorzugt weisen beiden Retraktorarme an ihren von dem Rahmen abstehenden Enden jeweils eine Befestigungseinrichtung, vorzugsweise eine hakenförmige Befestigungseinrichtung, zur Befestigung der Retraktorblätter an den Retraktorarmen auf. Die Retraktorblätter sowie die Retraktorarme sind vorzugsweise derart ausgestaltet, dass eine Befestigung der Retraktorblätter an den Retraktorarmen mittels eines Verriegelungsmechanismus, insbesondere eines sogenannten "Ball-snap"-Mechanismus, erfolgt. Der in diesem Absatz beschriebene Retraktor eignet sich insbesondere für die lumbale Wirbelsäule.

Bei dem im vorherigen Absatz beschriebenen Retraktor können grundsätzlich alle oder nur einzelne Retraktorkomponenten das mechanophore Material aufweisen oder aus diesem Material bestehen. Beispielsweise können insbesondere der Rahmen, wenigstens einer der beiden Retraktorarme und/oder wenigstens eines der beiden Retraktorblätter das mechanophore Material aufweisen oder aus dem mechanophoren Material bestehen. Erfindungsgemäß ist es jedoch bevorzugt, wenn lediglich die beiden Retraktorblätter oder wenigstens eines der beiden Retraktorblätter das mechanophore Material aufweist oder aus diesem Material besteht.

In einer alternativen Ausführungsform handelt es sich bei dem Retraktor um ein gebogenes Instrument mit einer gewölbten Auflagefläche, wobei das Instrument distalseitig eine Spitze, beispielsweise eine scharfe Spitze, stumpfe Spitze, kurze Spitze, V-Spitze, U-Spitze oder eine Schenkelhals-Spitze, aufweist.

In einer weiteren Ausführungsform (nicht erfindungsgemäß) handelt es sich bei dem Medizinprodukt um ein Knochenersatzmaterial, insbesondere um einen Knochenzement Bei dieser Ausführungsform kann das Medizinprodukt beispielsweise ferner Polymethylmethacrylat als Polymer aufweisen. Polymethylmethacrylat ist in der Gelenkendoprothetik insbesondere zur Verankerung von Implantaten am Knochen verwendbar. Bevorzugt bilden das Polymethylmethacrylat und das Mechanophor eine Mischung des Medizinprodukts. Das Mechanophor kann mit besonderem Vorteil eine Überlastung des Knochenersatzmaterials, insbesondere des Knochenzements, anzeigen. Dadurch können aufwendige Einfärbungen des Knochenersatzmaterials unterbleiben. Zusätzlich können Spannungen und Dehnungen sichtbar gemacht werden, welche noch nicht zu Rissen im Knochenersatzmaterial, insbesondere Knochenzement, geführt haben. Erfindungsgemäß kann es weiterhin vorgesehen sein, dass das Knochenersatzmaterial vor seiner Implantation ex vivo auf seine Belastbarkeit, insbesondere mechanische Belastbarkeit, überprüft wird. Hierzu kann beispielsweise das Mechanophor in Laborversuchen einem Knochenzementmaterial hinzugegeben werden.

Beschrieben wird die Verwendung eines Mechanophors für ein Medizinprodukt, vorzugsweise Probeimplantat oder Probeimplantatteil, oder zur Herstellung eines Medizinprodukts, vorzugsweise Probeimplantats oder Probeimplantatteils. Bezüglich weiterer Merkmale und Vorteile, insbesondere des Polymers, des Mechanophors, des Probeimplantats oder Probeimplantatteils, sowie des Medizinprodukts, wird zur Vermeidung von unnötigen Wiederholungen vollständig auf die im Rahmen des ersten Erfindungsaspekts gemachten Ausführungen Bezug genommen.

Weitere Merkmale und Vorteile der Erfindung ergeben sich aus der nachfolgenden Beschreibung bevorzugter Ausführungsformen in Form von Ausführungsbeispielen, den dazugehörigen Figuren sowie den Ansprüchen. Dabei können Merkmale jeweils für sich alleine oder in Kombination miteinander verwirklicht sein. Die nachfolgend beschriebenen Ausführungsformen dienen lediglich zur Erläuterung und zum besseren Verständnis der Erfindung und sind in keiner Weise einschränkend zu verstehen.

### FIGURENKURZBESCHREIBUNG

In den Figuren ist schematisch folgendes gezeigt:
- Fig. 1: eine Ausführungsform eines erfindungsgemäßen Medizinprodukts in Form eines Zwischenwirbelprobeimplantats,
- Fig. 2a bis 2c: weitere Ausführungsformen eines erfindungsgemäßen Medizinprodukts jeweils in Form eines Probeimplantats,
- Fig. 3: eine weitere Ausführungsform (nicht erfindungsgemäß) eines Medizinprodukts in Form eines chirurgischen Instruments mit einem Festkörpergelenk,
- Fig. 4a bis 4f: verschiedene Ausführungsformen (nicht erfindungsgemäß) eines Medizinprodukts jeweils in Form eines Festkörpergelenks,
- Fig. 5: eine weitere Ausführungsform (nicht erfindungsgemäß) eines Medizinprodukts in Form eines Retraktors und
- Fig. 6: eine weitere Ausführungsform (nicht erfindungsgemäß) eines Medizinprodukts in Form eines Retraktors.

### AUSFÜHRLICHE FIGURENBESCHREIBUNG

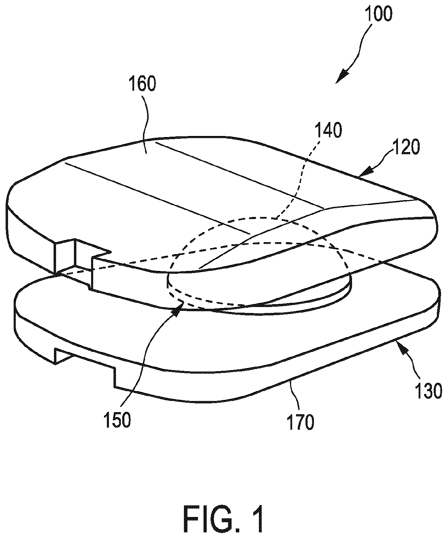

Fig. 1 zeigt schematisch eine Ausführungsform eines erfindungsgemäßen Medizinprodukts in Form eines Zwischenwirbelprobeimplantats 100.

Das Zwischenwirbelprobeimplantat 100 weist ein oberes Anlageelement 120 und ein unteres Anlageelement 130 auf. Die beiden Anlageelemente 120, 130 sind vorzugsweise annähernd plattenförmig ausgebildet. Beide Anlageelemente 120, 130 liegen an Lagerflächen flächig aneinander an. Dazu weist das untere Anlageelement 130 eine konvexe, kugelige, nach oben abstehende Lagerfläche 140 auf, welche in eine komplementäre, konkave Lagerfläche 150 an der Unterseite des oberen Anlageelementes 120 eintaucht. Die beiden Anlageelemente 120, 130 sind dadurch gegeneinander verschwenkbar, so dass ihre im Wesentlichen parallel verlaufenden Außenseiten 160, 170 in ihrer Neigung zueinander verstellbar sind. Die Außenseiten 160, 170 bilden Anlageflächen an benachbarte Wirbelkörper, wenn das Zwischenwirbelprobeimplantat 100 in einen Zwischenwirbelraum zwischen den beiden Wirbelkörpern eingeschoben ist (nicht dargestellt).

Zur Beurteilung der Kräfte, welche auf ein Zwischenwirbelimplantat in vivo wirken, weist das Zwischenwirbelprobeimplantat 100 ein Mechanophor auf.

Grundsätzlich können alle Komponenten oder nur einzelne Komponenten des Zwischenwirbelprobeimplantats 100, wie beispielsweise das obere Anlageelement 120, die Lagerfläche 150, das untere Anlageelement 130 und/oder die Lagerfläche 140, ein Mechanophor aufweisen.

Das Mechanophor ist vorzugsweise Bestandteil einer Mischung, wobei die Mischung neben dem Mechanophor insbesondere ein Polymer, wie beispielsweise Polymethylmethacrylat, enthalten kann. Bei der Mischung kann es sich insbesondere um eine Beschichtung des Zwischenwirbelprobeimplantats handeln.

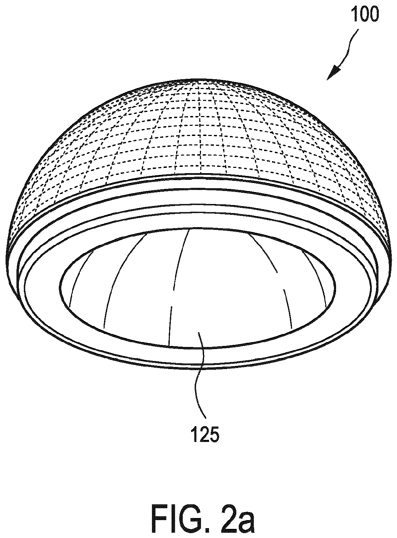

Fig. 2a zeigt eine weitere Ausführungsform eines erfindungsgemäßen Medizinprodukts in Form eines Probeimplantats 100.

Das Probeimplantat 100 ist als Probegelenkpfanne, insbesondere Probehüftgelenkpfanne, mit einer Gleitfläche 125 ausgestaltet.

Grundsätzlich können alle Bereiche des Probeimplantats 100 oder nur einzelne Bereiche davon ein Mechanophor, insbesondere eine Mischung mit einem Mechanophor oder eine Beschichtung mit einem Mechanophor, aufweisen. Erfindungsgemäß ist es bevorzugt, wenn lediglich die Gleitfläche 125 der Probegelenkpfanne 100 ein Mechanophor aufweist, insbesondere mit einer Beschichtung, welche ein Mechanophor enthält, versehen ist.

Die in Fig. 2a dargestellte Probegelenkpfanne dient der Beurteilung von Kräften, welche auf eine künstliche Gelenkpfanne, insbesondere künstliche Hüftgelenkpfanne, in vivo wirken.

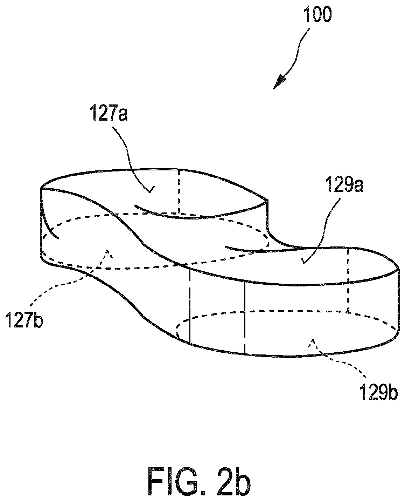

Fig. 2b zeigt eine weitere Ausführungsform eines erfindungsgemäßen Medizinprodukts in Form eines Probeimplantats 100.

Das Probeimplantat 100 besitzt zwei Artikulationsflächen 127a, 129a zum Bewirken einer Artikulation mit einem Femur sowie zwei Artikulationsflächen 127b und 129b zum Bewirken einer Artikulation mit einer Tibia.

Die Artikulationsflächen 127a; 129a sowie die Artikulationsflächen 127b; 129b können jeweils gleich ausgestaltet sein (sogenanntes symmetrisches Meniskusprobeimplantat). Alternativ können die Artikulationsflächen 127a; 129a bzw. die Artikulationsflächen 127b; 129b jeweils unterschiedlich ausgestaltet sein (sogenanntes asymmetrisches Meniskusprobeimplantat).

Das Probeimplantat 100 eignet sich zur Beurteilung von Kräften, welche auf ein künstliches Meniskusimplantat in vivo wirken.

Grundsätzlich können alle Bereiche oder nur einzelne Bereiche des Probeimplantats 100 ein Mechanophor aufweisen, insbesondere mit einer ein Mechanophor enthaltenden Beschichtung versehen sein. Erfindungsgemäß ist es bevorzugt, wenn nur die Artikulationsflächen 127a; 129a; 127b; 129b ein Mechanophor aufweisen, insbesondere mit einer ein Mechanophor enthaltenden Beschichtung versehen sind. Dadurch ist eine Beurteilung möglich, ob die in vivo auf die Artikulationsflächen 127a; 129a; 127b; 129b einwirkenden Kräfte gleich groß sind.

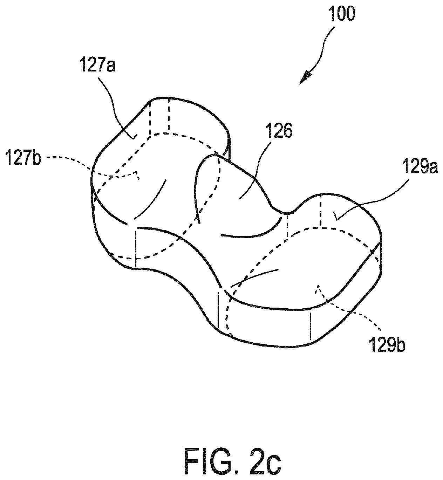

Fig. 2c zeigt eine weitere Ausführungsform eines erfindungsgemäßen Medizinprodukts in Form eines Probeimplantats 100.

Das Probeimplantat 100 weist zwei Artikulationsflächen 127a, 129a zum Bewirken einer Artikulation mit einem Femur sowie zwei Artikulationsflächen 127b; 129b zum Bewirken einer Artikulation mit einer Tibia auf.

Die Artikulationsflächen 127a; 129a sowie die Artikulationsflächen 127b; 129b können jeweils gleich ausgestaltet sein (sogenanntes symmetrisches Meniskusprobeimplantat). Alternativ können die Artikulationsflächen 127a; 129a bzw. die Artikulationsflächen 127b; 129b jeweils unterschiedlich ausgestaltet sein (sogenanntes asymmetrisches Meniskusprobeimplantat). Weiterhin weist das Probeimplantat einen Zapfen ("Post") 126 auf. Der Zapfen ist ebenfalls für eine Artikulation mit einem Femur gestaltet. Hierzu muss der Zapfen 126 Translationskräfte aufnehmen können. Der Zapfen 126 ist zwischen den Artikulationsflächen 127a; 129a angeordnet.

Auch das in Fig. 2c dargestellte Probeimplantat 100 dient zur Beurteilung von Kräften, welche auf ein künstliches Meniskusimplantat in vivo einwirken.

Grundsätzlich können alle Bereiche oder nur einzelne Bereiche des Probeimplantats 100 ein Mechanophor aufweisen, insbesondere mit einer ein Mechanophor enthaltenden Beschichtung versehen sein.

Erfindungsgemäß ist es bevorzugt, wenn lediglich die Artikulationsflächen 127a; 129a; 127b; 129b und/oder der Zapfen 126 ein Mechanophor aufweisen, insbesondere mit einer Beschichtung, welche ein Mechanophor enthält, versehen sind. Dadurch ist eine Beurteilung möglich, ob die in vivo auf die Artikulationsflächen 127a; 129a; 127b; 129b einwirkenden Kräfte gleich groß sind und/oder in vivo einwirkende Kräfte unterhalb eines Grenzwertes bleiben, dessen Überschreiten ein Verformen oder Zerstören, insbesondere Brechen, des Zapfens 126 zur Folge hätte.

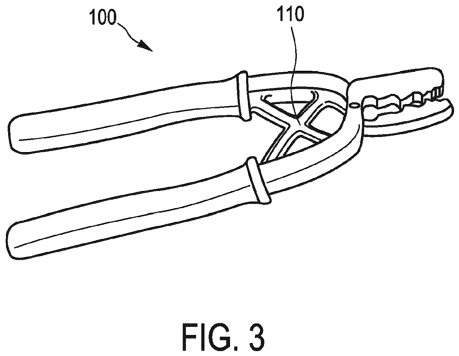

Fig. 3 zeigt eine Ausführungsform (nicht erfindungsgemäß) eines Medizinprodukts in Form eines medizinischen Instruments 100. Das Instrument 100 ist als Greifzange mit einem wagenradförmigen Festkörpergelenk 110 ausgeführt. Das Festkörpergelenk 110 weist ein Mechanophor auf. Das Festkörpergelenk 110 kann insbesondere mit einer Beschichtung versehen sein, welche ein Mechanophor enthält. Auf diese Weise kann mit besonderem Vorteil eine Art "Kraftanzeige" realisiert werden. Eine derartige Kraftanzeige kann beispielsweise bei der Ermittlung von Gelenkspalten im Rahmen einer knieendoprothetischen Versorgung von Vorteil sein, um bei der Aufspreizung der Streck- und Beugespalte im medialen und lateralen Kompartement des Gelenks immer die gleiche Kraft aufzubringen.

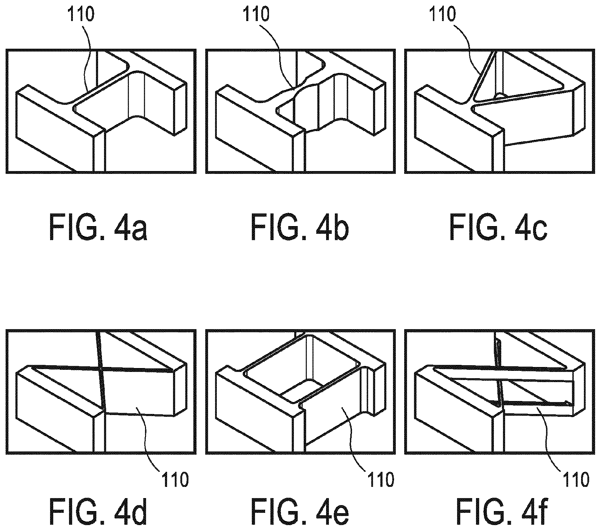

Die Fig. 4a bis 4f zeigen jeweils eine Ausführungsform (nicht erfindungsgemäß) eines Medizinprodukts jeweils in Form eines Festkörpergelenks 110.

Bei dem in Fig. 4a dargestellten Festkörpergelenk 110 handelt es sich um ein Blattfedergelenk.

Bei dem in Fig. 4b dargestellten Festkörpergelenk 110 handelt es sich um ein Kerbgelenk.

Bei dem in Fig. 4c dargestellten Festkörpergelenk 110 handelt es sich um ein Dreieckgelenk.

Bei dem in Fig. 4d dargestellten Festkörpergelenk 110 handelt es sich um ein Wagenradgelenk.

Bei dem in Fig. 4e dargestellten Festkörpergelenk 110 handelt es sich um ein Parallelgelenk.

Bei dem in Fig. 4f dargestellten Festkörpergelenk 110 handelt es sich um ein Kreuzfedergelenk.

Die in den Fig. 4a bis 4f gezeigten Festkörpergelenke 110 weisen jeweils ein Mechanophor auf. Insbesondere können die in den Fig. 4a bis 4f gezeigten Festkörpergelenke jeweils mit einer Beschichtung versehen sein, welche ein Mechanophor enthält.

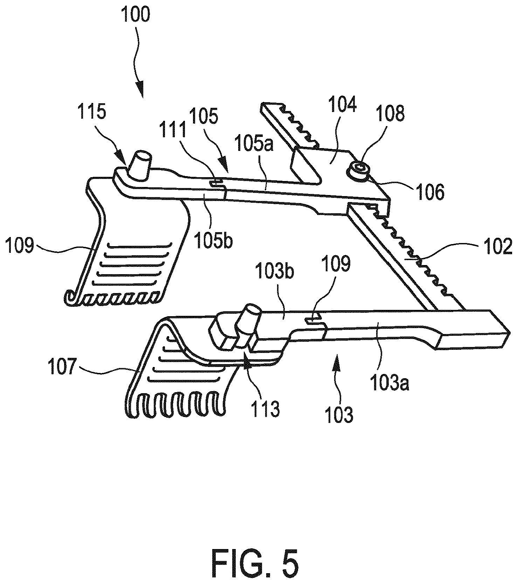

Fig. 5 zeigt eine weitere Ausführungsform (nicht erfindungsgemäß) eines Medizinprodukts in Form eines Retraktors 100.

Der Retraktor 100 weist einen Rahmen 102, zwei Retraktorarme 103, 105 sowie zwei Retraktorblätter 107, 109 auf.

Der Rahmen 102 liegt in Form einer geradlinigen Zahnstange vor.

Die Zahnstange 102 ist an einem Ende mit dem Retraktorarm 103 verbunden, vorzugsweise verschiebefest. Der Retraktorarm 103 steht senkrecht von der Zahnstange 102 ab.

Auf der Zahnstange 102 ist über eine Führungshülse 104 der zweite Retraktorarm 105 längs der Zahnstange 102 verschiebbar gelagert. Der Retraktorarm 105 verläuft parallel zum Retraktorarm 103 und hat dieselbe Länge wie der Retraktorarm 103.

Zur Fixierung des Retraktorarms 105 in einem definierten Abstand zu dem Retraktorarm 103 weist die Führungshülse 104 eine Stellschraube 106 auf. Die Stellschraube 106 weist an ihrer Außenseite einen Innensechskant 108 zur Aufnahme eines Drehwerkzeuges auf.

Der Retraktorarm 103 ist in zwei Abschnitte 103a, 103b untergliedert, welche über ein Gelenk 109 miteinander verbunden sind. Entsprechend ist der Retraktorarm 105 in zwei Abschnitte 105a, 105b untergliedert, welche über ein Gelenk 111 miteinander verbunden sind.

Die beiden Retraktorarme 103, 105 weisen an ihren von der Zahnstange 102 abstehenden Enden jeweils eine hakenförmige Befestigungseinrichtung 113, 115 zur Befestigung der Retraktorblätter 107, 109 auf. Bevorzugt sind die Retraktorblätter 107, 109 sowie die Retraktorarme 103, 105 derart ausgestaltet, dass eine Befestigung der Retraktorblätter 107, 109 an den Retraktorarmen 103, 105 mittels eines "Ball-snap"-Mechanismus erfolgt.

Um Geweberetraktionskräfte zu begrenzen, ist es bevorzugt, wenn wenigstens eines der beiden Retraktorblätter 107, 109, vorzugsweise beide Retraktorblätter 107, 109, ein Mechanophor aufweisen, insbesondere mit einer Beschichtung versehen sind, welche ein Mechanophor enthält.

Der in Fig. 5 dargestellte Retraktor eignet sich insbesondere für die lumbale Wirbelsäule.

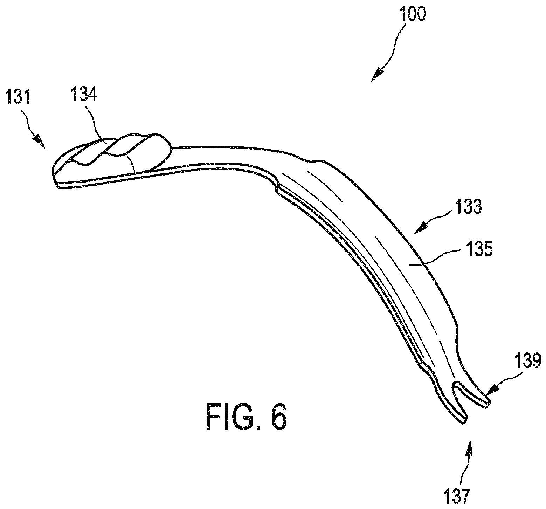

Fig. 6 zeigt eine weitere Ausführungsform (nicht erfindungsgemäß) eines Medizinprodukts 100 in Form eines Retraktors.

Der Retraktor 100 weist einen Abschnitt 133 mit einer gewölbten Auflagefläche 135 auf. Der Abschnitt 133 ist wenigstens abschnittsweise gebogen ausgebildet. Abhängig von der Stärke der Biegung des Abschnitts 133 kann der Retraktor für die Darstellung eines Acetabulums oder für die Darstellung eines Femurs verwendet werden.

Der Abschnitt 133 mündet an einem distalen Ende 137 des Retraktors 100 in eine U-Spitze 139. Zur Erleichterung der Handhabung kann der Retraktor an einem proximalen Ende 131 einen Handgriff 134 aufweisen. Alternativ kann der Retraktor an seinem proximalen Ende 131 einen nicht gebogenen, insbesondere gerade verlaufenden, Abschnitt aufweisen, welcher die Funktion eines Handgriffs übernimmt (nicht dargestellt).

Der Retraktor 100 kann insbesondere im Bereich seiner U-Spitze 139 ein Mechanophor aufweisen, insbesondere mit einer Beschichtung versehen sein, welche ein Mechanophor enthält. Bezüglich weiterer Merkmale und Vorteile des in den Figuren dargestellten Medizinprodukts, insbesondere des Mechanophors, wird vollständig auf die vorliegende Beschreibung Bezug genommen.

### AUSFÜHRUNGSBEISPIELE

### 1. Herstellung eines Probe-Kniegelenk-Inlays

Eine Mischung aus Polymethylmethacrylat und 3-(4-methoxyphenyl)-3-(3-methyl-4-methoxyphenyl)-13-hydroxy-indeno[2,1-f]naphto[1,2-b]pyran wurde aufgeschmolzen und in eine Form gegossen, welche dem herzustellenden Probe-Kniegelenk-Inlay entsprach. Nach Aushärtung der Mischung wurde das Probe-Inlay entformt.

### 2. Herstellung einer Probe-Hüftgelenksgleitfläche

Eine Mischung aus Polymethylmethacrylat und Piroxicam wurde aufgeschmolzen und in einen Quader entsprechender Größe gegossen. Nach Aushärtung der Mischung wurde anschließend aus dem Vollkörper mit Hilfe einer CNC-Fräse eine Probe-Hüftgelenksgleitfläche gefräst.

### 3. Herstellung eines Festkörpergelenks (nicht erfindungsgemäß)

Eine Mischung aus Polymethylmethacrylat und 3-(4-methoxyphenyl)-3-(3-methyl-4-methoxyphenyl)-13-hydroxy-indeno[2,1-f]naphto[1,2-b]pyran wurde aufgeschmolzen und in einen Quader entsprechender Größer gegossen. Nach Aushärtung der Mischung wurde aus dem Vollkörper mit Hilfe einer CNC-Fräse ein Festkörpergelenk gefräst, welches anschließend in ein Spreizinstrument eingesetzt wurde.

## Patentansprüche

1. Medizinprodukt enthaltend ein Mechanophor, wobei es sich bei dem Medizinprodukt um ein Implantat oder Implantatteil handelt, **dadurch gekennzeichnet, dass** es sich bei dem Implantat oder Implantatteil um eine Gelenkprothese, ein Teil einer Gelenkprothese, ein Probeimplantat oder ein Probeimplantatteil handelt.

2. Medizinprodukt nach Anspruch 1, **dadurch gekennzeichnet, dass** das Mechanophor Bestandteil einer Mischung oder Zusammensetzung des Medizinprodukts ist.

3. Medizinprodukt nach Anspruch 1 oder 2, **dadurch gekennzeichnet, dass** das Medizinprodukt, insbesondere die Mischung bzw. Zusammensetzung, ferner ein Polymer aufweist.

4. Medizinprodukt nach Anspruch 3, **dadurch gekennzeichnet, dass** das Polymer ausgewählt ist aus der Gruppe, aufweisend Polyolefine, Polyethylen (PE), ultrahochmolekulares Polyethylen (UHMWPE), Polyvinylchlorid (PVC), Polyvinylidenchlorid, Polyvinylfluorid, Polyvinylidenfluorid, Polytetrafluorethylen (PTFE), Polystyrol, Polyvinylalkohol (PVA), Polyacrylsäure oder Polyacrylat, Polymethacrylsäure oder Polymethacrylat, Polymethylmethacrylsäure oder Polymethylmethacrylat, Polydimethylsiloxan, Polyurethane, amorphe Polymere, Elastomere, thermoplastische Elastomere, Polypropylen, Polytetrafluorpropylen, Polyhexafluorpropylen, Polyethylenglykol, Polypropylenglykol, Polyester, Polyethylenterephthalsäure oder Polyethylenterephthalat, Polypropylenterephthalsäure oder Polypropylenterephthalat, Polybutylenterephthalsäure oder Polybutylenterephthalat, Polyhydroxyalkansäuren oder Polyhydroxyalkanoate, Polymilchsäure bzw. Polylactid, Polyglykolsäure oder Polyglykolid, Polyhydroxybuttersäure oder Polyhydroxybutyrat, Poly-3-hydroxybuttersäure oderPoly-3-hydroxybutyrat, Poly-4-hydroxybuttersäure oder Poly-4-hydroxybutyrat, Polytrimethylencarbonat, Poly-e-caprolacton, Poly-para-Dioxanon, Copolymere und Mischungen, insbesondere Blends, davon.

5. Medizinprodukt nach Anspruch 3 oder 4, **dadurch gekennzeichnet, dass** das Mechanophor nicht kovalent mit dem Polymer verknüpft ist.

6. Medizinprodukt nach einem der vorhergehenden Ansprüche, **dadurch gekennzeichnet, dass** es sich bei dem Mechanophor um eine mechanochrome Verbindung handelt, d.h. um eine Verbindung, welche bei Belastung eine farbverursachende oder farbverändernde Strukturänderung erfährt.

7. Medizinprodukt nach Anspruch 6, **dadurch gekennzeichnet, dass** die Strukturänderung mit einer Ringöffnung des Mechanophors einhergeht.

8. Medizinprodukt nach Anspruch 6 oder 7, **dadurch gekennzeichnet, dass** die Strukturänderung reversibel ist, insbesondere unter Einwirkung von Licht, vorzugsweise UV-Licht.

9. Medizinprodukt nach einem der vorhergehenden Ansprüche, **dadurch gekennzeichnet, dass** das Mechanophor ausgewählt ist aus der Gruppe aufweisend Pyrane, Oxazine, Fulgide, Fulgimide, Diarylethylene, dimere Lactone, dimere Imidazole und Mischungen davon.

10. Medizinprodukt nach einem der vorhergehenden Ansprüche, **dadurch gekennzeichnet, dass** die Gelenkprothese ausgewählt ist aus der Gruppe aufweisend Kniegelenkprothese, Hüftgelenkprothese, Knöchelgelenkprothese, Schultergelenkprothese, Kiefergelenkprothese, Ellenbogengelenkprothese, Fingergelenkprothese und Wirbelsäulengelenkflächenprothese.

## Claims

1. Medical product containing a mechanophore, wherein the medical product is an implant or implant part, **characterized in that** the implant or implant part is a joint prosthesis, a part of a joint prosthesis, a trial implant or a trial implant part.

2. Medical product according to claim 1, **characterized in that** the mechanophore is a component of a mixture or composition of the medical product.

3. Medical product according to claim 1 or 2, **characterized in that** the medical product, in particular the mixture or composition, further includes a polymer.

4. Medical product according to claim 3, **characterized in that** the polymer is selected from the group comprising polyolefins, polyethylene (PE), ultrahigh molecular weight polyethylene (UHMWPE), polyvinyl chloride (PVC), polyvinylidene chloride, polyvinyl fluoride, polyvinylidene fluoride, polytetrafluorethylene (PTFE), polystyrene, polyvinyl alcohol (PVA), polyacrylic acid or polyacrylate, polymethacrylic acid or polymethacrylate, polymethylmethacrylic acid or polymethyl methacrylate, polydimethylsiloxane, polyurethanes, amorphous polymers, elastomers, thermoplastic elastomers, polypropylene, polytetrafluoropropylene, polyhexafluoropropylene, polyethylene glycol, polypropylene glycol, polyester, polyethylene terephthalic acid or polyethylene terephthalate, polypropylene terephthalic acid or polypropylene terephthalate, polybutylene terephthalic acid or polybutylene terephthalate, polyhydroxyalkanoic acids or polyhydroxyalkanoates, polylactic acid or polylactide, polyglycolic acid or polyglycolide, polyhydroxybutyric acid or polyhydroxybutyrate, poly-3-hydroxybutyric acid or poly-3-hydroxybutyrate, poly-4-hydroxybutyric acid or poly-4-hydroxybutyrate, polytrimethylene carbonate, poly-e-caprolactone, poly-para-dioxanone, copolymers and mixtures, in particular blends, thereof.

5. Medical product according to claim 3 or 4, **characterized in that** the mechanophore is not covalently linked to the polymer.

6. Medical product according to any of the preceding claims, **characterized in that** the mechanophore is a mechanochromic compound, i.e. a compound which under stress undergoes a color-producing or color-changing structural change.

7. Medical product according to claim 6, **characterized in that** the structural change is accompanied by ring opening of the mechanophore.

8. Medical product according to claim 6 or 7, **characterized in that** the structural change is reversible, in particular under the action of light, preferably UV light.

9. Medical product according to any of the preceding claims, **characterized in that** the mechanophore is selected from the group comprising pyrans, oxazines, fulgides, fulgimides, diarylethylenes, dimeric lactones, dimeric imidazoles and mixtures thereof.

10. Medical product according to any of the preceding claims, **characterized in that** the joint prosthesis is selected from the group comprising knee joint prosthesis, hip joint prosthesis, ankle joint prosthesis, shoulder joint prosthesis, mandibular joint prosthesis, elbow joint prosthesis, finger joint prosthesis and spinal facet prosthesis.

## Revendications

1. Produit médical contenant un mécanophore, le produit médical consistant en un implant ou une partie d'implant, **caractérisé en ce que** l'implant ou la partie d'implant consiste en une prothèse d'articulation, une partie d'une prothèse d'articulation, un implant d'essai ou une partie d'implant d'essai.

2. Produit médical selon la revendication 1, **caractérisé en ce que** le mécanophore est un constituant d'un mélange ou d'une composition du produit médical.

3. Produit médical selon la revendication 1 ou 2, **caractérisé en ce que** le produit médical, notamment le mélange ou la composition, comprend en outre un polymère.

4. Produit médical selon la revendication 3, **caractérisé en ce que** le polymère est choisi dans le groupe comprenant les polyoléfines, le polyéthylène (PE), le polyéthylène de poids moléculaire ultra-élevé (UHMWPE), le polychlorure de vinyle (PVC), le polychlorure de vinylidène, le polyfluorure de vinyle, le polyfluorure de vinylidène, le polytétrafluoroéthylène (PTFE), le polystyrène, l'alcool polyvinylique (PVA), l'acide polyacrylique ou le polyacrylate, l'acide polyméthacrylique ou le polyméthacrylate, l'acide polyméthylméthacrylique, ou le polyméthacrylate de méthyle, le polydiméthylsiloxane, les polyuréthanes, les polymères amorphes, les élastomères, les élastomères thermoplastiques, le polypropylène, le polytétrafluoropropylène, le polyhexafluoropropylène, le polyéthylène glycol, le polypropylène glycol, les polyesters, l'acide polyéthylène-téréphtalique ou le polytéréphtalate d'éthylène, l'acide polypropylène-téréphtalique ou le polytéréphtalate de propylène, l'acide polybutylène-téréphtalique ou le polytéréphtalate de butylène, les acides polyhydroxyalcanoïques ou les polyhydroxyalcanoates, l'acide polylactique ou le polylactide, l'acide polyglycolique ou le polyglycolide, l'acide polyhydroxybutyrique ou le polyhydroxybutyrate, l'acide poly-3-hydroxybutyrique ou le poly-3-hydroxybutyrate, l'acide poly-4-hydroxybutyrique ou le poly-4-hydroxybutyrate, le polycarbonate de triméthylène, la poly-ε-caprolactone, la poly-para-dioxanone, les copolymères et les mélanges, notamment les mélanges homogènes, de ceux-ci.

5. Produit médical selon la revendication 3 ou 4, **caractérisé en ce que** le mécanophore n'est pas relié de manière covalente avec le polymère.

6. Produit médical selon l'une quelconque des revendications précédentes, **caractérisé en ce que** le mécanophore consiste en un composé mécanochrome, c.-à-d. un composé qui subit une modification structurale provoquant une coloration ou changeant une coloration lors d'une sollicitation.

7. Produit médical selon la revendication 6, **caractérisé en ce que** la modification structurale est accompagnée d'une ouverture de cycle du mécanophore.

8. Produit médical selon la revendication 6 ou 7, **caractérisé en ce que** la modification structurale est réversible, notamment sous l'action de lumière, de préférence de lumière UV.

9. Produit médical selon l'une quelconque des revendications précédentes, **caractérisé en ce que** le mécanophore est choisi dans le groupe comprenant les pyranes, les oxazines, les fulgides, les fulgimides, les diaryléthylènes, les lactones dimères, les imidazoles dimères et leurs mélanges.

10. Produit médical selon l'une quelconque des revendications précédentes, **caractérisé en ce que** la prothèse d'articulation est choisie dans le groupe comprenant les prothèses d'articulation du genou, les prothèses d'articulation de la hanche, les prothèses d'articulation de la cheville, les prothèses d'articulation de l'épaule, les prothèses d'articulation de la mâchoire, les prothèses d'articulation du coude, les prothèses d'articulation du doigt et les prothèses de surfaces d'articulations vertébrales.
